# EUROPEAN PATENT SPECIFICATION

(11) **EP 1 496 737 B1**
(45) Date of publication and mention of the grant of the patent: **11.07.2007**
(21) Application number: 03719841.3
(22) Date of filing: 01.05.2003
(51) Int. Cl.: A01H 1/00, A01H 5/00, C12N 15/29, C12N 15/82

(54) **.CE1, A REGULATOR OF COLD INDUCED TRANSCRIPTOME AND FREEZING TOLERANCE IN PLANTS**
ICE1, EIN REGULATOR DES KÄLTEINDUZIERTEN TRANSCRIPTOMS UND DER GEFRIERTOLERANZ BEI PFLANZEN
ICE1, UN REGULATEUR DE TRANSCRIPTOME INDUIT PAR LE FROID ET DE LA TOLERANCE AU GEL CHEZ LES PLANTES

(30) Priority: 01.05.2002 US 377469 P; 02.05.2002 US 377897 P
(43) Date of publication of application: 19.01.2005
(73) Proprietor: The Arizona Board of Regents on behalf of the University of Arizona, Tucson, AZ 85721-0158 (US)
(72) Inventor: ZHU, Jian-Kang, Tucson, AZ 85719 (US); CHINNUSAMY, Viswanathan, Tucson, AZ 85719 (US); OHTA, Masaru, Tsukuba, Ibaraki 305-0031 (JP); KANRAR, Siddharta, Tucson, AZ 85719 (US); LEE, Byeong-ha, Tucson, AZ 85712 (US); AGARWAL, Manu, Tucson, AZ 85719 (US)
(74) Representative: Poulin, Gérard
(86) International application number: PCT/US2003/012118
(87) International publication number: WO 2003/093411

(56) References cited:
- WO-A-02/15675
- WO-A-03/013227
- GILMOUR S J ET AL: "Low temperature regulation of the Arabidopsis CBF family of AP2 transcriptional activators as an early step in cold -induced COR gene expression" PLANT JOURNAL, BLACKWELL SCIENTIFIC PUBLICATIONS, OXFORD, GB, vol. 16, no. 4, November 1998 (1998-11), pages 433-442, XP002108959 ISSN: 0960-7412
- VISWANATHAN C. ET AL: 'ICE1: a regulator of cold-induced transcriptome and freezing tolerance in Arabidopsis' GENES AND DEVELOPMENT vol. 17, 2003, pages 1043 - 1054, XP002978717
- JAGLO K.R. ET AL: 'Components of the Arabidopsis C-Repeat/Dehydration-Responsive Element Binding Factor Cold-Response Pathway Are Conserved in Brassica napus and Other Plant Species' PLANT PHYSIOLOGY vol. 127, November 2001, pages 910 - 917, XP002978718
- GILMOUR SJ ET AL: 'Overexpression of the Arabidopsis CBF3 transcriptional activator mimics multiplebiochemical changes associated with cold acclimation' PLANT PHYSIOL. vol. 124, no. 4, December 2000, pages 1854 - 1865, XP002978719

### SEQUENCE LISTING

<110> THE ARIZONA BOARD OF REGENTS ON BEHALF OF THE UNIVERSITY OF ARIZONA
<120> ICE1, A REGULATOR OF COLD INDUCED TRANSCRIPTOME AND FREEZING TOLERANCE IN PLANTS
<130> SR 25998.US/EE
<140> EP n°03 719 841.3
   <141> 2003-05-01
<150> 60/377,469
   <151> 2002-05-01
<150> 60/377,897
   <151> 2002-05-02
<160> 42
<170> PatentIn version 3.1
<210> 1
   <211> 2021
   <212> DNA
   <213> Arabidopsis thaliania
<400> 1
   atcaaaaaaa aagtttcaat ttttgaaagc tctgagaaat gaatctatca ttctctctct    60
   ctatctctat cttccttttc agatttcgct tcttcaattc atgaaatcct cgtgattcta   120
   ctttaatgct tctctttttt tacttttcca agtctctgaa tattcaaagt atatatcttt   180
   tgttttcaaa cttttgcaga attgtcttca agcttccaaa tttcagttaa aggtctcaac   240
   tttgcagaat tttcctctaa aggttcagac tttggggtaa aggtgtcaac tttggcgatg   300
   ggtcttgacg gaaacaatgg tggaggggtt tggttaaacg gtggtggtgg agaaagggaa   360
   gagaacgagg aaggttcatg gggaaggaat caagaagatg gttcttctca gtttaagcct   420
   atgcttgaag gtgattggtt tagtagtaac caaccacatc cacaagatct tcagatgtta   480
   cagaatcagc cagatttcag atactttggt ggttttcctt ttaaccctaa tgataatctt   540
   cttcttcaac actctattga ttcttcttct tcttgttctc cttctcaagc ttttagtctt   600
   gacccttctc agcaaaatca gttcttgtca actaacaaca acaagggttg tcttctcaat   660
   gttccttctt ctgcaaaccc ttttgataat gcttttgagt ttggctctga atctggtttt   720
   cttaaccaaa tccatgctcc tatttcgatg gggtttggtt ctttgacaca attggggaac   780
   agggatttga gttctgttcc tgatttcttg tctgctcggt cacttcttgc gccggaaagc   840
   aacaacaaca acacaatgtt gtgtggtggt ttcacagctc cgttggagtt ggaaggtttt   900
   ggtagtcctg ctaatggtgg ttttgttggg aacagagcga aagttctgaa gcctttagag   960
   gtgttagcat cgtctggtgc acagcctact ctgttccaga aacgtgcagc tatgcgtcag  1020
   agctctggaa gcaaaatggg aaattcggag agttcgggaa tgaggaggtt tagtgatgat  1080
   ggagatatgg atgagactgg gattgaggtt tctgggttga actatgagtc tgatgagata  1140
   aatgagagcg gtaaagcggc tgagagtgtt cagattggag gaggaggaaa gggtaagaag  1200
   aaaggtatgc ctgctaagaa tctgatggct gagaggagaa ggaggaagaa gcttaatgat  1260
   aggctttata tgcttagatc agttgtcccc aagatcagca aaatggatag agcatcaata  1320
   cttggagatg caattgatta tctgaaggaa cttctacaaa ggatcaatga tcttcacaat  1380
   gaacttgagt caactcctcc tggatctttg cctccaactt catcaagctt ccatccgttg  1440
   acacctacac cgcaaactct ttcttgtcgt gtcaaggaag agttgtgtcc ctcttcttta  1500
   ccaagtccta aaggccagca agctagagtt gaggttagat taagggaagg aagagcagtg  1560
   aacattcata tgttctgtgg tcgtagaccg ggtctgttgc tcgctaccat gaaagctttg  1620
   gataatcttg gattggatgt tcagcaagct gtgatcagct gttttaatgg gtttgccttg  1680
   gatgttttcc gcgctgagca atgccaagaa ggacaagaga tactgcctga tcaaatcaaa  1740
   gcagtgcttt tcgatacagc agggtatgct ggtatgatct gatctgatcc tgacttcgag  1800
   tccattaagc atctgttgaa gcagagctag aagaactaag tccctttaaa tctgcaattt  1860
   tcttctcaac tttttttctt atgtcataac ttcaatctaa gcatgtaatg caattgcaaa  1920
   tgagagttgt ttttaaatta agcttttgag aacttgaggt tgttgttgtt ggatacataa  1980
   cttcaacctt ttattagcaa tgttaacttc catttatgtc t                      2021
<210> 2
   <211> 494
   <212> PRT
   <213> Arabidopsis thaliania
<400> 2
   Met Gly Leu Asp Gly Asn Asn Gly Gly Gly Val Trp Leu Asn Gly Gly
   1               5                   10                  15
   Gly Gly Glu Arg Glu Glu Asn Glu Glu Gly Ser Trp Gly Arg Asn Gln
               20                  25                  30
   Glu Asp Gly Ser Ser Gln Phe Lys Pro Met Leu Glu Gly Asp Trp Phe
           35                  40                  45
   Ser Ser Asn Gln Pro His Pro Gln Asp Leu Gln Met Leu Gln Asn Gln
       50                  55                  60
   Pro Asp Phe Arg Tyr Phe Gly Gly Phe Pro Phe Asn Pro Asn Asp Asn
   65                  70                  75                  80
   Leu Leu Leu Gln His Ser Ile Asp Ser Ser Ser Ser Cys Ser Pro Ser
                   85                  90                  95
   Gln Ala Phe Ser Leu Asp Pro Ser Gln Gln Asn Gln Phe Leu Ser Thr
               100                 105                 110
   Asn Asn Asn Lys Gly Cys Leu Leu Asn Val Pro Ser Ser Ala Asn Pro
           115                 120                 125
   Phe Asp Asn Ala Phe Glu Phe Gly Ser Glu Ser Gly Phe Leu Asn Gln
   130 135 140
   Ile His Ala Pro Ile Ser Met Gly Phe Gly Ser Leu Thr Gln Leu Gly
   145 150 155 160
   Asn Arg Asp Leu Ser Ser Val Pro Asp Phe Leu Ser Ala Arg Ser Leu
   165 170 175
   Leu Ala Pro Glu Ser Asn Asn Asn Asn Thr Met Leu Cys Gly Gly Phe
   180 185 190
   Thr Ala Pro Leu Glu Leu Glu Gly Phe Gly Ser Pro Ala Asn Gly Gly
   195 200 205
   Phe Val Gly Asn Arg Ala Lys Val Leu Lys Pro Leu Glu Val Leu Ala
   210 215 220
   Ser Ser Gly Ala Gln Pro Thr Leu Phe Gln Lys Arg Ala Ala Met Arg
   225 230 235 240
   Gln Ser Ser Gly Ser Lys Met Gly Asn Ser Glu Ser Ser Gly Met Arg
   245 250 255
   Arg Phe Ser Asp Asp Gly Asp Met Asp Glu Thr Gly Ile Glu Val Ser
   260 265 270
   Gly Leu Asn Tyr Glu Ser Asp Glu Ile Asn Glu Ser Gly Lys Ala Ala
   275 280 285
   Glu Ser Val Gln Ile Gly Gly Gly Gly Lys Gly Lys Lys Lys Gly Met
   290 295 300
   Pro Ala Lys Asn Leu Met Ala Glu Arg Arg Arg Arg Lys Lys Leu Asn
   305 310 315 320
   Asp Arg Leu Tyr Met Leu Arg Ser Val Val Pro Lys Ile Ser Lys Met
   325 330 335
   Asp Arg Ala Ser Ile Leu Gly Asp Ala Ile Asp Tyr Leu Lys Glu Leu
   340 345 350
   Leu Gln Arg Ile Asn Asp Leu His Asn Glu Leu Glu Ser Thr Pro Pro
   355 360 365
   Gly Ser Leu Pro Pro Thr Ser Ser Ser Phe His Pro Leu Thr Pro Thr
   370 375 380
   Pro Gln Thr Leu Ser Cys Arg Val Lys Glu Glu Leu Cys Pro Ser Ser
   385 390 395 400
   Leu Pro Ser Pro Lys Gly Gln Gln Ala Arg Val Glu Val Arg Leu Arg
   405 410 415
   Glu Gly Arg Ala Val Asn Ile His Met Phe Cys Gly Arg Arg Pro Gly
   420 425 430
   Leu Leu Leu Ala Thr Met Lys Ala Leu Asp Asn Leu Gly Leu Asp Val
   435 440 445
   Gln Gln Ala Val Ile Ser Cys Phe Asn Gly Phe Ala Leu Asp Val Phe
   450 455 460
   Arg Ala Glu Gln Cys Gln Glu Gly Gln Glu Ile Leu Pro Asp Gln Ile
   465 470 475 480
   Lys Ala Val Leu Phe Asp Thr Ala Gly Tyr Ala Gly Met Ile
   485 490
<210> 3
   <211> 828
   <212> PRT
   <213> Arabidopsis thaliania
<400> 3
   Met Glu Ser Arg Glu Asp Ser Phe Ile Ser Lys Glu Lys Lys Ser Thr
   1               5                   10                  15
   Met Lys Lys Glu Lys Gln Ala Ile Ala Ser Gln Arg Asn Arg Arg Arg
               20                  25                  30
   Val Ile Lys Asn Arg Gly Asn Gly Lys Arg Leu Ile Ala Ser Leu Ser
           35                  40                  45
   Gln Arg Lys Arg Arg Arg Ile Pro Arg Gly Arg Gly Asn Glu Lys Ala
       50                  55                  60
   Val Phe Ala Pro Ser Ser Leu Pro Asn Asp Val Val Glu Glu Ile Phe
   65                  70                  75                  80
   Leu Arg Leu Pro Val Lys Ala Ile Ile Gln Leu Lys Ser Leu Ser Lys
                   85                  90                  95
   Gln Trp Arg Ser Thr Ile Glu Ser Arg Ser Phe Glu Glu Arg His Leu
               100                 105                 110
   Lys Ile Val Glu Arg Ser Arg Val Asp Phe Pro Gln Val Met Val Met
           115                 120                 125
   Ser Glu Glu Tyr Ser Leu Lys Gly Ser Lys Gly Asn Gln Pro Arg Pro
       130                 135                 140
   Asp Thr Asp Ile Gly Phe Ser Thr Ile Cys Leu Glu Ser Ala Ser Ile
   145 150 155 160
   Leu Ser Ser Thr Leu Ile Thr Phe Pro Gln Gly Phe Gln His Arg Ile
   165 170 175
   Tyr Ala Ser Glu Ser Cys Asp Gly Leu Phe Cys Ile His Ser Leu Lys
   180 185 190
   Thr Gln Ala Ile Tyr Val Val Asn Pro Ala Thr Arg Trp Phe Arg Gln
   195 200 205
   Leu Pro Pro Ala Arg Phe Gln Ile Leu Met Gln Lys Leu Tyr Pro Thr
   210 215 220
   Gln Asp Thr Trp Ile Asp Ile Lys Pro Val Val Cys Tyr Thr Ala Phe
   225 230 235 240
   Val Lys Ala Asn Asp Tyr Lys Leu Val Trp Leu Tyr Asn Ser Asp Ala
   245 250 255
   Ser Asn Pro Asn Leu Gly Val Thr Lys Cys Glu Val Phe Asp Phe Arg
   260 265 270
   Ala Asn Ala Trp Arg Tyr Leu Thr Cys Thr Pro Ser Tyr Arg Ile Phe
   275 280 285
   Pro Asp Gln Val Pro Ala Ala Thr Asn Gly Ser Ile Tyr Trp Phe Thr
   290 295 300
   Glu Pro Tyr Asn Gly Glu Ile Lys Val Val Ala Leu Asp Ile His Thr
   305 310 315 320
   Glu Thr Phe Arg Val Leu Pro Lys Ile Asn Pro Ala Ile Ala Ser Ser
   325 330 335
   Asp Pro Asp His Ile Asp Met Cys Thr Leu Asp Asn Gly Leu Cys Met
   340 345 350
   Ser Lys Arg Glu Ser Asp Thr Leu Val Gln Glu Ile Trp Arg Leu Lys
   355 360 365
   Ser Ser Glu Asp Ser Trp Glu Lys Phe Asp Met Asn Ser Asp Gly Val
   370 375 380
   Trp Leu Asp Gly Ser Gly Glu Ser Pro Glu Val Asn Asn Gly Glu Ala
   385 390 395 400
   Ala Ser Trp Val Arg Asn Pro Asp Glu Asp Trp Phe Asn Asn Pro Pro
   405 410 415
   Pro Pro Gln His Thr Asn Gln Asn Asp Phe Arg Phe Asn Gly Gly Phe
   420 425 430
   Pro Leu Asn Pro Ser Glu Asn Leu Leu Leu Leu Leu Gln Gln Ser Ile
   435 440 445
   Asp Ser Ser Ser Ser Ser Ser Pro Leu Leu His Pro Phe Thr Leu Asp
   450 455 460
   Ala Ala Ser Gln Gln Gln Gln Gln Gln Gln Gln Gln Gln Glu Gln Ser
   465 470 475 480
   Phe Leu Ala Thr Lys Ala Cys Ile Val Ser Leu Leu Asn Val Pro Thr
   485 490 495
   Ile Asn Asn Asn Thr Phe Asp Asp Phe Gly Phe Asp Ser Gly Phe Leu
   500 505 510
   Gly Gln Gln Phe His Gly Asn His Gln Ser Pro Asn Ser Met Asn Phe
   515 520 525
   Thr Gly Leu Asn His Ser Val Pro Asp Phe Leu Pro Ala Pro Glu Asn
   530 535 540
   Ser Ser Gly Ser Cys Gly Leu Ser Pro Leu Phe Ser Asn Arg Ala Lys
   545 550 555 560
   Val Leu Lys Pro Leu Gln Val Met Ala Ser Ser Gly Ser Gln Pro Thr
   565 570 575
   Leu Phe Gln Lys Arg Ala Ala Met Arg Gln Ser Ser Ser Ser Lys Met
   580 585 590
   Cys Asn Ser Glu Ser Ser Ser Glu Met Arg Lys Ser Ser Tyr Glu Arg
   595 600 605
   Glu Ile Asp Asp Thr Ser Thr Gly Ile Ile Asp Ile Ser Gly Leu Asn
   610 615 620
   Tyr Glu Ser Asp Asp His Asn Thr Asn Asn Asn Lys Gly Lys Lys Lys
   625 630 635 640
   Gly Met Pro Ala Lys Asn Leu Met Ala Glu Arg Arg Arg Arg Lys Lys
   645 650 655
   Leu Asn Asp Arg Leu Tyr Met Leu Arg Ser Val Val Pro Lys Ile Ser
   660 665 670
   Lys Met Asp Arg Ala Ser Ile Leu Gly Asp Ala Ile Asp Tyr Leu Lys
   675 680 685
   Glu Leu Leu Gln Arg Ile Asn Asp Leu His Thr Glu Leu Glu Ser Thr
   690 695 700
   Pro Pro Ser Ser Ser Ser Leu His Pro Leu Thr Pro Thr Pro Gln Thr
   705 710 715 720
   Leu Ser Tyr Arg Val Lys Glu Glu Leu Cys Pro Ser Ser Ser Leu Pro
   725 730 735
   Ser Pro Lys Gly Gln Gln Pro Arg Val Glu Val Arg Leu Arg Glu Gly
   740 745 750
   Lys Ala Val Asn Ile His Met Phe Cys Gly Arg Arg Pro Gly Leu Leu
   755 760 765
   Leu Ser Thr Met Arg Ala Leu Asp Asn Leu Gly Leu Asp Val Gln Gln
   770 775 780
   Ala Val Ile Ser Cys Phe Asn Gly Phe Ala Leu Asp Val Phe Arg Ala
   785 790 795 800
   Glu Gln Cys Gln Glu Asp His Asp Val Leu Pro Glu Gln Ile Lys Ala
   805 810 815
   Val Leu Leu Asp Thr Ala Gly Tyr Ala Gly Leu Val
   820 825
<210> 4
   <211> 351
   <212> PRT
   <213> Arabidopsis thaliania
<400> 4
   Met Glu Leu Ser Thr Gln Met Asn Val Phe Glu Glu Leu Leu Val Pro
   1               5                   10                  15
   Thr Lys Gln Glu Thr Thr Asp Asn Asn Ile Asn Asn Leu Ser Phe Asn
               20                  25                  30
   Gly Gly Phe Asp His His His His Gln Phe Phe Pro Asn Gly Tyr Asn
           35                  40                  45
   Ile Asp Tyr Leu Cys Phe Asn Asn Glu Glu Glu Asp Glu Asn Thr Leu
       50                  55                  60
   Leu Tyr Pro Ser Ser Phe Met Asp Leu Ile Ser Gln Pro Pro Pro Leu
   65                  70                  75                  80
   Leu Leu His Gln Pro Pro Pro Leu Gln Pro Leu Ser Pro Pro Leu Ser
                   85                  90                  95
   Ser Ser Ala Thr Ala Gly Ala Thr Phe Asp Tyr Pro Phe Leu Glu Ala
   100 105 110
   Leu Gln Glu Ile Ile Asp Ser Ser Ser Ser Ser Pro Pro Leu Ile Leu
   115 120 125
   Gln Asn Gly Gln Glu Glu Asn Phe Asn Asn Pro Met Ser Tyr Pro Ser
   130 135 140
   Pro Leu Met Glu Ser Asp Gln Ser Lys Ser Phe Ser Val Gly Tyr Cys
   145 150 155 160
   Gly Gly Glu Thr Asn Lys Lys Lys Ser Lys Lys Leu Glu Gly Gln Pro
   165 170 175
   Ser Lys Asn Leu Met Ala Glu Arg Arg Arg Arg Lys Arg Leu Asn Asp
   180 185 190
   Arg Leu Ser Met Leu Arg Ser Ile Val Pro Lys Ile Ser Lys Met Asp
   195 200 205
   Arg Thr Ser Ile Leu Gly Asp Ala Ile Asp Tyr Met Lys Glu Leu Leu
   210 215 220
   Asp Lys Ile Asn Lys Leu Gln Asp Glu Glu Gln Glu Leu Gly Asn Ser
   225 230 235 240
   Asn Asn Ser His His Ser Lys Leu Phe Gly Asp Leu Lys Asp Leu Asn
   245 250 255
   Ala Asn Glu Pro Leu Val Arg Asn Ser Pro Lys Phe Glu Ile Asp Arg
   260 265 270
   Arg Asp Glu Asp Thr Arg Val Asp Ile Cys Cys Ser Pro Lys Pro Gly
   275 280 285
   Leu Leu Leu Ser Thr Val Asn Thr Leu Glu Thr Leu Gly Leu Glu Ile
   290 295 300
   Glu Gln Cys Val Ile Ser Cys Phe Ser Asp Phe Ser Leu Gln Ala Ser
   305 310 315 320
   Cys Ser Glu Gly Ala Glu Gln Arg Asp Phe Ile Thr Ser Glu Asp Ile
   325 330 335
   Lys Gln Ala Leu Phe Arg Asn Ala Gly Tyr Gly Gly Ser Cys Leu
   340 345 350
<210> 5
   <211> 315
   <212> PRT
   <213> Arabidopsis thaliania
<400> 5
   Met Glu Thr Glu Leu Thr Gln Leu Arg Lys Gln Glu Ser Asn Asn Leu
   1               5                   10                  15
   Asn Gly Val Asn Gly Gly Phe Met Ala Ile Asp Gln Phe Val Pro Asn
               20                  25                  30
   Asp Trp Asn Phe Asp Tyr Leu Cys Phe Asn Asn Leu Leu Gln Glu Asp
           35                  40                  45
   Asp Asn Ile Asp His Pro Ser Ser Ser Ser Leu Met Asn Leu Ile Ser
       50                  55                  60
   Gln Pro Pro Pro Leu Leu His Gln Pro Pro Gln Pro Ser Ser Pro Leu
   65                  70                  75                  80
   Tyr Asp Ser Pro Pro Leu Ser Ser Ala Phe Asp Tyr Pro Phe Leu Glu
                   85                  90                  95
   Asp Ile Ile His Ser Ser Tyr Ser Pro Pro Pro Leu Ile Leu Pro Ala
               100                 105                 110
   Ser Gln Glu Asn Thr Asn Asn Tyr Ser Pro Leu Met Glu Glu Ser Lys
           115                 120                 125
   Ser Phe Ile Ser Ile Gly Glu Thr Asn Lys Lys Arg Ser Asn Lys Lys
       130                 135                 140
   Leu Glu Gly Gln Pro Ser Lys Asn Leu Met Ala Glu Arg Arg Arg Arg
   145                 150                 155                 160
   Lys Arg Leu Asn Asp Arg Leu Ser Leu Leu Arg Ser Ile Val Pro Lys
                   165                 170                 175
   Ile Thr Lys Met Asp Arg Thr Ser Ile Leu Gly Asp Ala Ile Asp Tyr
               180                 185                 190
   Met Lys Glu Leu Leu Asp Lys Ile Asn Lys Leu Gln Glu Asp Glu Gln
           195                 200                 205
   Glu Leu Gly Ser Asn Ser His Leu Ser Thr Leu Ile Thr Asn Glu Ser
       210                 215                 220
   Met Val Arg Asn Ser Leu Lys Phe Glu Val Asp Gln Arg Glu Val Asn
   225                 230                 235                 240
   Thr His Ile Asp Ile Cys Cys Pro Thr Lys Pro Gly Leu Val Val Ser
                   245                 250                 255
   Thr Val Ser Thr Leu Glu Thr Leu Gly Leu Glu Ile Glu Gln Cys Val
               260                 265                 270
   Ile Ser Cys Phe Ser Asp Phe Ser Leu Gln Ala Ser Cys Phe Glu Val
           275                 280                 285
   Gly Glu Gln Arg Tyr Met Val Thr Ser Glu Ala Thr Lys Gln Ala Leu
       290                 295                 300
   Ile Arg Asn Ala Gly Tyr Gly Gly Arg Cys Leu
   305                 310                 315
<210> 6
   <211> 623
   <212> PRT
   <213> Arabidopsis thaliania
<400> 6
   Met Thr Asp Tyr Arg Leu Gln Pro Thr Met Asn Leu Trp Thr Thr Asp
   1               5                   10                  15
   Asp Asn Ala Ser Met Met Glu Ala Phe Met Ser Ser Ser Asp Ile Ser
               20                  25                  30
   Thr Leu Trp Pro Pro Ala Ser Thr Thr Thr Thr Thr Ala Thr Thr Glu
           35                  40                  45
   Thr Thr Pro Thr Pro Ala Met Glu Ile Pro Ala Gln Ala Gly Phe Asn
       50                  55                  60
   Gln Glu Thr Leu Gln Gln Arg Leu Gln Ala Leu Ile Glu Gly Thr His
   65                  70                  75                  80
   Glu Gly Trp Thr Tyr Ala Ile Phe Trp Gln Pro Ser Tyr Asp Phe Ser
                   85                  90                  95
   Gly Ala Ser Val Leu Gly Trp Gly Asp Gly Tyr Tyr Lys Gly Glu Glu
               100                 105                 110
   Asp Lys Ala Asn Pro Arg Arg Arg Ser Ser Ser Pro Pro Phe Ser Thr
           115                 120                 125
   Pro Ala Asp Gln Glu Tyr Arg Lys Lys Val Leu Arg Glu Leu Asn Ser
       130                 135                 140
   Leu Ile Ser Gly Gly Val Ala Pro Ser Asp Asp Ala Val Asp Glu Glu
   145                 150                 155                 160
   Val Thr Asp Thr Glu Trp Phe Phe Leu Val Ser Met Thr Gln Ser Phe
                   165                 170                 175
   Ala Cys Gly Ala Gly Leu Ala Gly Lys Ala Phe Ala Thr Gly Asn Ala
               180                 185                 190
   Val Trp Val Ser Gly Ser Asp Gln Leu Ser Gly Ser Gly Cys Glu Arg
   195 200 205
   Ala Lys Gln Gly Gly Val Phe Gly Met His Thr Ile Ala Cys Ile Pro
   210 215 220
   Ser Ala Asn Gly Val Val Glu Val Gly Ser Thr Glu Pro Ile Arg Gln
   225 230 235 240
   Ser Ser Asp Leu Ile Asn Lys Val Arg Ile Leu Phe Asn Phe Asp Gly
   245 250 255
   Gly Ala Gly Asp Leu Ser Gly Leu Asn Trp Asn Leu Asp Pro Asp Gln
   260 265 270
   Gly Glu Asn Asp Pro Ser Met Trp Ile Asn Asp Pro Ile Gly Thr Pro
   275 280 285
   Gly Ser Asn Glu Pro Gly Asn Gly Ala Pro Ser Ser Ser Ser Gln Leu
   290 295 300
   Phe Ser Lys Ser Ile Gln Phe Glu Asn Gly Ser Ser Ser Thr Ile Thr
   305 310 315 320
   Glu Asn Pro Asn Leu Asp Pro Thr Pro Ser Pro Val His Ser Gln Thr
   325 330 335
   Gln Asn Pro Lys Phe Asn Asn Thr Phe Ser Arg Glu Leu Asn Phe Ser
   340 345 350
   Thr Ser Ser Ser Thr Leu Val Lys Pro Arg Ser Gly Glu Ile Leu Asn
   355 360 365
   Phe Gly Asp Glu Gly Lys Arg Ser Ser Gly Asn Pro Asp Pro Ser Ser
   370 375 380
   Tyr Ser Gly Gln Thr Gln Phe Glu Asn Lys Arg Lys Arg Ser Met Val
   385 390 395 400
   Leu Asn Glu Asp Lys Val Leu Ser Phe Gly Asp Lys Thr Ala Gly Glu
   405 410 415
   Ser Asp His Ser Asp Leu Glu Ala Ser Val Val Lys Glu Val Ala Val
   420 425 430
   Glu Lys Arg Pro Lys Lys Arg Gly Arg Lys Pro Ala Asn Gly Arg Glu
   435 440 445
   Glu Pro Leu Asn His Val Glu Ala Glu Arg Gln Arg Arg Glu Lys Leu
   450 455 460
   Asn Gln Arg Phe Tyr Ala Leu Arg Ala Val Val Pro Asn Val Ser Lys
   465 470 475 480
   Met Asp Lys Ala Ser Leu Leu Gly Asp Ala Ile Ala Tyr Ile Asn Glu
   485 490 495
   Leu Lys Ser Lys Val Val Lys Thr Glu Ser Glu Lys Leu Gln Ile Lys
   500 505 510
   Asn Gln Leu Glu Glu Val Lys Leu Glu Leu Ala Gly Arg Lys Ala Ser
   515 520 525
   Ala Ser Gly Gly Asp Met Ser Ser Ser Cys Ser Ser Ile Lys Pro Val
   530 535 540
   Gly Met Glu Ile Glu Val Lys Ile Ile Gly Trp Asp Ala Met Ile Arg
   545 550 555 560
   Val Glu Ser Ser Lys Arg Asn His Pro Ala Ala Arg Leu Met Ser Ala
   565 570 575
   Leu Met Asp Leu Glu Leu Glu Val Asn His Ala Ser Met Ser Val Val
   580 585 590
   Asn Asp Leu Met Ile Gln Gln Ala Thr Val Lys Met Gly Phe Arg Ile
   595 600 605
   Tyr Thr Gln Glu Gln Leu Arg Ala Ser Leu Ile Ser Lys Ile Gly
   610 615 620
<210> 7
   <211> 592
   <212> PRT
   <213> Arabidopsis thaliania
<400> 7
   Met Asn Gly Thr Thr Ser Ser Ile Asn Phe Leu Thr Ser Asp Asp Asp
   1 5 10 15
   Ala Ser Ala Ala Ala Met Glu Ala Phe Ile Gly Thr Asn His His Ser
   20 25 30
   Ser Leu Phe Pro Pro Pro Pro Gln Gln Pro Pro Gln Pro Gln Phe Asn
   35 40 45
   Glu Asp Thr Leu Gln Gln Arg Leu Gln Ala Leu Ile Glu Ser Ala Gly
   50 55 60
   Glu Asn Trp Thr Tyr Ala Ile Phe Trp Gln Ile Ser His Asp Phe Asp
   65 70 75 80
   Ser Ser Thr Gly Asp Asn Thr Val Ile Leu Gly Trp Gly Asp Gly Tyr
   85 90 95
   Tyr Lys Gly Glu Glu Asp Lys Glu Lys Lys Lys Asn Asn Thr Asn Thr
   100 105 110
   Ala Glu Gln Glu His Arg Lys Arg Val Ile Arg Glu Leu Asn Ser Leu
   115 120 125
   Ile Ser Gly Gly Ile Gly Val Ser Asp Glu Ser Asn Asp Glu Glu Val
   130 135 140
   Thr Asp Thr Glu Trp Phe Phe Leu Val Ser Met Thr Gln Ser Phe Val
   145 150 155 160
   Asn Gly Val Gly Leu Pro Gly Glu Ser Phe Leu Asn Ser Arg Val Ile
   165 170 175
   Trp Leu Ser Gly Ser Gly Ala Leu Thr Gly Ser Gly Cys Glu Arg Ala
   180 185 190
   Gly Gln Gly Gln Ile Tyr Gly Leu Lys Thr Met Val Cys Ile Ala Thr
   195 200 205
   Gln Asn Gly Val Val Glu Leu Gly Ser Ser Glu Val Ile Ser Gln Ser
   210 215 220
   Ser Asp Leu Met His Lys Val Asn Asn Leu Phe Asn Phe Asn Asn Gly
   225 230 235 240
   Gly Gly Asn Asn Gly Val Glu Ala Ser Ser Trp Gly Phe Asn Leu Asn
   245 250 255
   Pro Asp Gln Gly Glu Asn Asp Pro Ala Leu Trp Ile Ser Glu Pro Thr
   260 265 270
   Asn Thr Gly Ile Glu Ser Pro Ala Arg Val Asn Asn Gly Asn Asn Ser
   275 280 285
   Asn Ser Asn Ser Lys Ser Asp Ser His Gln Ile Ser Lys Leu Glu Lys
   290 295 300
   Asn Asp Ile Ser Ser Val Glu Asn Gln Asn Arg Gln Ser Ser Cys Leu
   305 310 315 320
   Val Glu Lys Asp Leu Thr Phe Gln Gly Gly Leu Leu Lys Ser Asn Glu
   325 330 335
   Thr Leu Ser Phe Cys Gly Asn Glu Ser Ser Lys Lys Arg Thr Ser Val
   340 345 350
   Ser Lys Gly Ser Asn Asn Asp Glu Gly Met Leu Ser Phe Ser Thr Val
   355 360 365
   Val Arg Ser Ala Ala Asn Asp Ser Asp His Ser Asp Leu Glu Ala Ser
   370 375 380
   Val Val Lys Glu Ala Ile Val Val Glu Pro Pro Glu Lys Lys Pro Arg
   385 390 395 400
   Lys Arg Gly Arg Lys Pro Ala Asn Gly Arg Glu Glu Pro Leu Asn His
   405 410 415
   Val Glu Ala Glu Arg Gln Arg Arg Glu Lys Leu Asn Gln Arg Phe Tyr
   420 425 430
   Ser Leu Arg Ala Val Val Pro Asn Val Ser Lys Met Asp Lys Ala Ser
   435 440 445
   Leu Leu Gly Asp Ala Ile Ser Tyr Ile Asn Glu Leu Lys Ser Lys Leu
   450 455 460
   Gln Gln Ala Glu Ser Asp Lys Glu Glu Ile Gln Lys Lys Leu Asp Gly
   465 470 475 480
   Met Ser Lys Glu Gly Asn Asn Gly Lys Gly Cys Gly Ser Arg Ala Lys
   485 490 495
   Glu Arg Lys Ser Ser Asn Gln Asp Ser Thr Ala Ser Ser Ile Glu Met
   500 505 510
   Glu Ile Asp Val Lys Ile Ile Gly Trp Asp Val Met Ile Arg Val Gln
   515 520 525
   Cys Gly Lys Lys Asp His Pro Gly Ala Arg Phe Met Glu Ala Leu Lys
   530 535 540
   Glu Leu Asp Leu Glu Val Asn His Ala Ser Leu Ser Val Val Asn Asp
   545 550 555 560
   Leu Met Ile Gln Gln Ala Thr Val Lys Met Gly Ser Gln Phe Phe Asn
   565 570 575
   His Asp Gln Leu Lys Val Ala Leu Met Thr Lys Val Gly Glu Asn Tyr
   580 585 590
<210> 8
   <211> 610
   <212> PRT
   <213> Zea mays
<400> 8
   Met Ala Leu Ser Ala Ser Arg Val Gln Gln Ala Glu Glu Leu Leu Gln
   1               5                   10                  15
   Arg Pro Ala Glu Arg Gln Leu Met Arg Ser Gln Leu Ala Ala Ala Ala
               20                  25                  30
   Arg Ser Ile Asn Trp Ser Tyr Ala Leu Phe Trp Ser Ile Ser Asp Thr
           35                  40                  45
   Gln Pro Gly Val Leu Thr Trp Thr Asp Gly Phe Tyr Asn Gly Glu Val
       50                  55                  60
   Lys Thr Arg Lys Ile Ser Asn Ser Val Glu Leu Thr Ser Asp Gln Leu
   65                  70                  75                  80
   Val Met Gln Arg Ser Asp Gln Leu Arg Glu Leu Tyr Glu Ala Leu Leu
                   85                  90                  95
   Ser Gly Glu Gly Asp Arg Arg Ala Ala Pro Ala Arg Pro Ala Gly Ser
               100                 105                 110
   Leu Ser Pro Glu Asp Leu Gly Asp Thr Glu Trp Tyr Tyr Val Val Ser
           115                 120                 125
   Met Thr Tyr Ala Phe Arg Pro Gly Gln Gly Leu Pro Gly Arg Ser Phe
       130                 135                 140
   Ala Ser Asp Glu His Val Trp Leu Cys Asn Ala His Leu Ala Gly Ser
   145                 150                 155                 160
   Lys Ala Phe Pro Arg Ala Leu Leu Ala Lys Ser Ala Ser Ile Gln Ser
                   165                 170                 175
   Ile Leu Cys Ile Pro Val Met Gly Gly Val Leu Glu Leu Gly Thr Thr
               180                 185                 190
   Asp Thr Val Pro Glu Ala Pro Asp Leu Val Ser Arg Ala Thr Ala Ala
           195                 200                 205
   Phe Trp Glu Pro Gln Cys Pro Ser Ser Ser Pro Ser Gly Arg Ala Asn
       210                 215                 220
   Glu Thr Gly Glu Ala Ala Ala Asp Asp Gly Thr Phe Ala Phe Glu Glu
   225                 230                 235                 240
   Leu Asp His Asn Asn Gly Met Asp Asp Ile Glu Ala Met Thr Ala Ala
                   245                 250                 255
   Gly Gly His Gly Gln Glu Glu Glu Leu Arg Leu Arg Glu Ala Glu Ala
               260                 265                 270
   Leu Ser Asp Asp Ala Ser Leu Glu His Ile Thr Lys Glu Ile Glu Glu
   275 280 285
   Phe Tyr Ser Leu Cys Asp Glu Met Asp Leu Gln Ala Leu Pro Leu Pro
   290 295 300
   Leu Glu Asp Gly Trp Thr Val Asp Ala Ser Asn Phe Glu Val Pro Cys
   305 310 315 320
   Ser Ser Pro Gln Pro Ala Pro Pro Pro Val Asp Arg Ala Thr Ala Asn
   325 330 335
   Val Ala Ala Asp Ala Ser Arg Ala Pro Val Tyr Gly Ser Arg Ala Thr
   340 345 350
   Ser Phe Met Ala Trp Thr Arg Ser Ser Gln Gln Ser Ser Cys Ser Asp
   355 360 365
   Asp Ala Ala Pro Ala Ala Val Val Pro Ala Ile Glu Glu Pro Gln Arg
   370 375 380
   Leu Leu Lys Lys Val Val Ala Gly Gly Gly Ala Trp Glu Ser Cys Gly
   385 390 395 400
   Gly Ala Thr Gly Ala Ala Gln Glu Met Ser Gly Thr Gly Thr Lys Asn
   405 410 415
   His Val Met Ser Glu Arg Lys Arg Arg Glu Lys Leu Asn Glu Met Phe
   420 425 430
   Leu Val Leu Lys Ser Leu Leu Pro Ser Ile His Arg Val Asn Lys Ala
   435 440 445
   Ser Ile Leu Ala Glu Thr Ile Ala Tyr Leu Lys Glu Leu Gln Arg Arg
   450 455 460
   Val Gln Glu Leu Glu Ser Ser Arg Glu Pro Ala Ser Arg Pro Ser Glu
   465 470 475 480
   Thr Thr Thr Arg Leu Ile Thr Arg Pro Ser Arg Gly Asn Asn Glu Ser
   485 490 495
   Val Arg Lys Glu Val Cys Ala Gly Ser Lys Arg Lys Ser Pro Glu Leu
   500 505 510
   Gly Arg Asp Asp Val Glu Arg Pro Pro Val Leu Thr Met Asp Ala Gly
   515 520 525
   Thr Ser Asn Val Thr Val Thr Val Ser Asp Lys Asp Val Leu Leu Glu
   530 535 540
   Val Gln Cys Arg Trp Glu Glu Leu Leu Met Thr Arg Val Phe Asp Ala
   545 550 555 560
   Ile Lys Ser Leu His Leu Asp Val Leu Ser Val Gln Ala Ser Ala Pro
   565 570 575
   Asp Gly Phe Met Gly Leu Lys Ile Arg Ala Gln Phe Ala Gly Ser Gly
   580 585 590
   Ala Val Val Pro Trp Met Ile Ser Glu Ala Leu Arg Lys Ala Ile Gly
   595 600 605
   Lys Arg
   610
<210> 9
   <211> 379
   <212> PRT
   <213> Arabidopsis thaliania
<400> 9
   Met Asp Glu Ser Ser Ile Ile Pro Ala Glu Lys Val Ala Gly Ala Glu
   1               5                   10                  15
   Lys Lys Glu Leu Gln Gly Leu Leu Lys Thr Ala Val Gln Ser Val Asp
               20                  25                  30
   Trp Thr Tyr Ser Val Phe Trp Gln Phe Cys Pro Gln Gln Arg Val Leu
           35                  40                  45
   Val Trp Gly Asn Gly Tyr Tyr Asn Gly Ala Ile Lys Thr Arg Lys Thr
       50                  55                  60
   Thr Gln Pro Ala Glu Val Thr Ala Glu Glu Ala Ala Leu Glu Arg Ser
   65                  70                  75                  80
   Gln Gln Leu Arg Glu Leu Tyr Glu Thr Leu Leu Ala Gly Glu Ser Thr
                   85                  90                  95
   Ser Glu Ala Arg Ala Cys Thr Ala Leu Ser Pro Glu Asp Leu Thr Glu
               100                 105                 110
   Thr Glu Trp Phe Tyr Leu Met Cys Val Ser Phe Ser Phe Pro Pro Pro
           115                 120                 125
   Ser Gly Met Pro Gly Lys Ala Tyr Ala Arg Arg Lys His Val Trp Leu
       130                 135                 140
   Ser Gly Ala Asn Glu Val Asp Ser Lys Thr Phe Ser Arg Ala Ile Leu
   145                 150                 155                 160
   Ala Lys Thr Val Val Cys Ile Pro Met Leu Asp Gly Val Val Glu Leu
                   165                 170                 175
   Gly Thr Thr Lys Lys Asn Gly Lys Glu His Gln Gln Val Lys Thr Ala
   180 185 190
   Pro Ser Ser Gln Trp Val Leu Lys Gln Met Ile Phe Arg Val Pro Phe
   195 200 205
   Leu His Asp Asn Thr Lys Asp Lys Arg Leu Pro Arg Glu Asp Leu Ser
   210 215 220
   His Val Val Ala Glu Arg Arg Arg Arg Glu Lys Leu Asn Glu Lys Phe
   225 230 235 240
   Ile Thr Leu Arg Ser Met Val Pro Phe Val Thr Lys Met Asp Lys Val
   245 250 255
   Ser Ile Leu Gly Asp Thr Ile Ala Tyr Val Asn His Leu Arg Lys Arg
   260 265 270
   Val His Glu Leu Glu Asn Thr His His Glu Gln Gln His Lys Arg Thr
   275 280 285
   Arg Thr Cys Lys Arg Lys Thr Ser Glu Glu Val Glu Val Ser Ile Ile
   290 295 300
   Glu Asn Asp Val Leu Leu Glu Met Arg Cys Glu Tyr Arg Asp Gly Leu
   305 310 315 320
   Leu Leu Asp Ile Leu Gln Val Leu His Glu Leu Gly Ile Glu Thr Thr
   325 330 335
   Ala Val His Thr Ser Val Asn Asp His Asp Phe Glu Ala Glu Ile Arg
   340 345 350
   Ala Lys Val Arg Gly Lys Lys Ala Ser Ile Ala Glu Val Lys Arg Ala
   355 360 365
   Ile His Gln Val Ile Ile His Asp Thr Asn Leu
   370 375
<210> 10
   <211> 432
   <212> PRT
   <213> Arabidopsis thaliania
<400> 10
   Met Glu Gln Val Phe Ala Asp Trp Asn Phe Glu Asp Asn Phe His Met
   1               5                   10                  15
   Ser Thr Asn Lys Arg Ser Ile Arg Pro Glu Asp Glu Leu Val Glu Leu
               20                  25                  30
   Leu Trp Arg Asp Gly Gln Val Val Leu Gln Ser Gln Ala Arg Arg Glu
   35 40 45
   Pro Ser Val Gln Val Gln Thr His Lys Gln Glu Thr Asn Gln Glu Thr
   50 55 60
   Val Gln Lys Pro Asn Tyr Ala Ala Leu Asp Asp Gln Glu Thr Val Ser
   65 70 75 80
   Trp Ile Gln Tyr Pro Pro Asp Asp Val Ile Asp Pro Phe Glu Ser Glu
   85 90 95
   Phe Ser Ser His Phe Phe Ser Ser Ile Asp His Leu Gly Gly Pro Glu
   100 105 110
   Lys Pro Arg Met Ile Glu Glu Thr Val Lys His Glu Ala Gln Ala Met
   115 120 125
   Ala Pro Pro Lys Phe Arg Ser Ser Val Ile Thr Val Gly Pro Ser His
   130 135 140
   Cys Gly Ser Asn Gln Ser Thr Asn Ile His Gln Ala Thr Thr Leu Pro
   145 150 155 160
   Val Ser Met Ser Asp Arg Ser Lys Asn Val Glu Glu Arg Leu Asp Thr
   165 170 175
   Ser Ser Gly Gly Ser Ser Gly Cys Ser Tyr Gly Arg Asn Asn Lys Glu
   180 185 190
   Thr Val Ser Gly Thr Ser Val Thr Ile Asp Arg Lys Arg Lys His Val
   195 200 205
   Met Asp Ala Asp Gln Glu Ser Val Ser Gln Ser Asp Ile Gly Leu Thr
   210 215 220
   Ser Thr Asp Asp Gln Thr Met Gly Asn Lys Ser Ser Gln Arg Ser Gly
   225 230 235 240
   Ser Thr Arg Arg Ser Arg Ala Ala Glu Val His Asn Leu Ser Glu Arg
   245 250 255
   Arg Arg Arg Asp Arg Ile Asn Glu Arg Met Lys Ala Leu Gln Glu Leu
   260 265 270
   Ile Pro His Cys Ser Arg Thr Asp Lys Ala Ser Ile Leu Asp Glu Ala
   275 280 285
   Ile Asp Tyr Leu Lys Ser Leu Gln Met Gln Leu Gln Val Met Trp Met
   290 295 300
   Gly Ser Gly Met Ala Ala Ala Ala Ala Ala Ala Ala Ser Pro Met Met
   305 310 315 320
   Phe Pro Gly Val Gln Ser Ser Pro Tyr Ile Asn Gln Met Ala Met Gln
   325 330 335
   Ser Gln Met Gln Leu Ser Gln Phe Pro Val Met Asn Arg Ser Ala Pro
   340 345 350
   Gln Asn His Pro Gly Leu Val Cys Leu Asn Pro Val Gln Leu Gln Leu
   355 360 365
   Gln Ala Gln Asn Gln Ile Leu Ser Glu Gln Leu Ala Arg Tyr Met Gly
   370 375 380
   Gly Ile Pro Gln Met Pro Pro Ala Gly Asn Gln Thr Val Gln Gln Gln
   385 390 395 400
   Pro Ala Asp Met Leu Gly Phe Gly Ser Pro Ala Gly Pro Gln Ser Gln
   405 410 415
   Leu Ser Ala Pro Ala Thr Thr Asp Ser Leu His Met Gly Lys Ile Gly
   420 425 430
<210> 11
   <211> 430
   <212> PRT
   <213> Arabidopsis thaliania
<400> 11
   Met Glu His Gln Gly Trp Ser Phe Glu Glu Asn Tyr Ser Leu Ser Thr
   1               5                   10                  15
   Asn Arg Arg Ser Ile Arg Pro Gln Asp Glu Leu Val Glu Leu Leu Trp
               20                  25                  30
   Arg Asp Gly Gln Val Val Leu Gln Ser Gln Thr His Arg Glu Gln Thr
           35                  40                  45
   Gln Thr Gln Lys Gln Asp His His Glu Glu Ala Leu Arg Ser Ser Thr
       50                  55                  60
   Phe Leu Glu Asp Gln Glu Thr Val Ser Trp Ile Gln Tyr Pro Pro Asp
   65                  70                  75                  80
   Glu Asp Pro Phe Glu Pro Asp Asp Phe Ser Ser His Phe Phe Ser Thr
                   85                  90                  95
   Met Asp Pro Leu Gln Arg Pro Thr Ser Glu Thr Val Lys Pro Lys Ser
               100                 105                 110
   Ser Pro Glu Pro Pro Gln Val Met Val Lys Pro Lys Ala Cys Pro Asp
   115 120 125
   Pro Pro Pro Gln Val Met Pro Pro Pro Lys Phe Arg Leu Thr Asn Ser
   130 135 140
   Ser Ser Gly Ile Arg Glu Thr Glu Met Glu Gln Tyr Ser Val Thr Thr
   145 150 155 160
   Val Gly Pro Ser His Cys Gly Ser Asn Pro Ser Gln Asn Asp Leu Asp
   165 170 175
   Val Ser Met Ser His Asp Arg Ser Lys Asn Ile Glu Glu Lys Leu Asn
   180 185 190
   Pro Asn Ala Ser Ser Ser Ser Gly Gly Ser Ser Gly Cys Ser Phe Gly
   195 200 205
   Lys Asp Ile Lys Glu Met Ala Ser Gly Arg Cys Ile Thr Thr Asp Arg
   210 215 220
   Lys Arg Lys Arg Ile Asn His Thr Asp Glu Ser Val Ser Leu Ser Asp
   225 230 235 240
   Ala Ile Gly Asn Lys Ser Asn Gln Arg Ser Gly Ser Asn Arg Arg Ser
   245 250 255
   Arg Ala Ala Glu Val His Asn Leu Ser Glu Arg Arg Arg Arg Asp Arg
   260 265 270
   Ile Asn Glu Arg Met Lys Ala Leu Gln Glu Leu Ile Pro His Cys Ser
   275 280 285
   Lys Thr Asp Lys Ala Ser Ile Leu Asp Glu Ala Ile Asp Tyr Leu Lys
   290 295 300
   Ser Leu Gln Leu Gln Leu Gln Val Met Trp Met Gly Ser Gly Met Ala
   305 310 315 320
   Ala Ala Ala Ala Ser Ala Pro Met Met Phe Pro Gly Val Gln Pro Gln
   325 330 335
   Gln Phe Ile Arg Gln Ile Gln Ser Pro Val Gln Leu Pro Arg Phe Pro
   340 345 350
   Val Met Asp Gln Ser Ala Ile Gln Asn Asn Pro Gly Leu Val Cys Gln
   355 360 365
   Asn Pro Val Gln Asn Gln Ile Ile Ser Asp Arg Phe Ala Arg Tyr Ile
   370 375 380
   Gly Gly Phe Pro His Met Gln Ala Ala Thr Gln Met Gln Pro Met Glu
   385 390 395 400
   Met Leu Arg Phe Ser Ser Pro Ala Gly Gln Gln Ser Gln Gln Pro Ser
   405 410 415
   Ser Val Pro Thr Lys Thr Thr Asp Gly Ser Arg Leu Asp His
   420 425 430
<210> 12
   <211> 165
   <212> PRT
   <213> Danio rerio
<400> 12
   Met Ser Asp Asn Asp Asp Ile Glu Val Asp Ser Asp Ala Asp Ser Pro
   1 5 10 15
   Arg Phe His Gly Val Ala Asp Lys Arg Ala His His Asn Ala Leu Glu
   20 25 30
   Arg Lys Arg Arg Asp His Ile Lys Asp Ser Phe His Ser Leu Arg Asp
   35 40 45
   Ser Val Pro Ala Leu Gln Gly Glu Lys Gln Ser Ile Lys Gln Ala Ser
   50 55 60
   Arg Ala Gln Ile Leu Asp Lys Ala Thr Glu Tyr Ile Gln Tyr Met Arg
   65 70 75 80
   Arg Lys Asn His Thr His Gln Gln Asp Ile Asp Asp Leu Lys Arg Gln
   85 90 95
   Asn Ala Leu Leu Glu Gln Gln Val Arg Ala Leu Glu Lys Val Lys Gly
   100 105 110
   Thr Thr Gln Leu Gln Ala Asn Tyr Ser Ser Ser Asp Ser Ser Leu Tyr
   115 120 125
   Thr Asn Pro Lys Gly Gln Ala Val Ser Ala Phe Asp Gly Gly Ser Asp
   130 135 140
   Ser Ser Ser Gly Ser Glu Pro Glu Glu Gln Arg Thr Arg Lys Lys His
   145 150 155 160
   Arg Pro Glu Asp Ser
   165
<210> 13
   <211> 440
   <212> PRT
   <213> Homo sapiens
<400> 13
<210> 14
   <211> 32
   <212> DNA
   <213> Artificial Sequence
<220>
   <223> Synthetic DNA
<400> 14
   tcatggatcc accatttgtt aatgcatgat gg 32
<210> 15
   <211> 26
   <212> DNA
   <213> Artificial sequence
<220>
   <223> Synthetic DNA
<400> 15
   gctcaagctt tctgttctag ttcagg 26
<210> 16
   <211> 24
   <212> DNA
   <213> Artificial Sequence
<220>
   <223> Synthetic DNA
<400> 16
   ttcgattttt atttccattt ttgg 24
<210> 17
   <211> 22
   <212> DNA
   <213> Artificial sequence
<220>
   <223> Synthetic DNA
<400> 17
   ccaaacgtcc ttgagtcttg at 22
<210> 18
   <211> 26
   <212> DNA
   <213> Artificial sequence
<220>
   <223> Synthetic DNA
<400> 18
   taaaactcag attattattt ccattt 26
<210> 19
   <211> 20
   <212> DNA
   <213> Artificial Sequence
<220>
   <223> Synthetic DNA
<400> 19
   gaggagccac gtagagggcc 20
<210> 20
   <211> 24
   <212> DNA
   <213> Artificial Sequence
<220>
   <223> Synthetic DNA
<400> 20
   cgtggatcac agcaatacag agcc 24
<210> 21
   <211> 25
   <212> DNA
   <213> Artificial Sequence
<220>
   <223> Synthetic DNA
<400> 21
   cctcctgcac ttccacttcg tcttc 25
<210> 22
   <211> 37
   <212> DNA
   <213> Artificial Sequence
<220>
   <223> synthetic DNA
<400> 22
   agggatccgg accaccgtca ataacatcgt taagtag 37
<210> 23
   <211> 40
   <212> DNA
   <213> Artificial sequence
<220>
   <223> Synthetic DNA
<400> 23
   cgaattctaa ccgccattaa ctatgtctcc tctctatctc 40
<210> 24
   <211> 37
   <212> DNA
   <213> Artificial Sequence
<220>
   <223> Synthetic DNA
<400> 24
   agggatccgg accaccgtca ataacatcgt taagtag 37
<210> 25
   <211> 34
   <212> DNA
   <213> Artificial sequence
<220>
   <223> Synthetic DNA
<400> 25
   cgaattcgcc aaagttgaca cctttacccc aaag 34
<210> 26
   <211> 28
   <212> DNA
   <213> Artificial sequence
<220>
   <223> synthetic DNA
<400> 26
   gcgatgggtc ttgacggaaa caatggtg 28
<210> 27
   <211> 31
   <212> DNA
   <213> Artificial Sequence
<220>
   <223> Synthetic DNA
<400> 27 31
   tcagatcata ccagcatacc ctgctgtatc g 31
<210> 28
   <211> 21
   <212> DNA
   <213> Artificial Sequence
<220>
   <223> synthetic DNA
<400> 28 21
   gtcaagaggt tctcagcagt a 21
<210> 29
   <211> 21
   <212> DNA
   <213> Artificial sequence
<220>
   <223> synthetic DNA
<400> 29
   tcaccttctt gatccgcagt t 21
<210> 30
   <211> 36
   <212> DNA
   <213> Artificial Sequence
<220>
   <223> Synthetic DNA
<400> 30
   gctctagagc gatgggtctt gacggaaaca atggtg 36
<210> 31
   <211> 39
   <212> DNA
   <213> Artificial Sequence
<220>
   <223> Synthetic DNA
<400> 31
   ggggtacctc agatcatacc agcataccct gctgtatcg 39
<210> 32
   <211> 36
   <212> DNA
   <213> Artificial Sequence
<220>
   <223> Synthetic DNA
<400> 32
   aggaattcgc gatgggtctt gacggaaaca atggtg 36
<210> 33
   <211> 39
   <212> DNA
   <213> Artificial Sequence
<220>
   <223> Synthetic DNA
<400> 33
   ctggatcctc agatcatacc agcataccct gctgtatcg 39
<210> 34
   <211> 20
   <212> DNA
   <213> Artificial Sequence
<220>
   <223> Synthetic DNA
<400> 34
   accccaccat ttgttaatgc 20
<210> 35
   <211> 20
   <212> DNA
   <213> Artificial Sequence
<220>
   <223> Synthetic DNA
<400> 35
   acaattacaa ctgcatgctt 20
<210> 36
   <211> 20
   <212> DNA
   <213> Artificial Sequence
<220>
   <223> Synthetic DNA
<400> 36
   aatgttacat ttgatcattc 20
<210> 37
   <211> 20
   <212> DNA
   <213> Artificial sequence
<220>
   <223> synthetic DNA
<400> 37
   ctctggacac atggcagatc 20
<210> 38
   <211> 20
   <212> DNA
   <213> Artificial sequence
<220>
   <223> synthetic DNA
   <400> 38
   cattttacaa ttgcttcgct 20
<210> 39
   <211> 20
   <212> DNA
   <213> Artificial sequence
<220>
   <223> synthetic DNA
<400> 39
   atataattaa ctacttttat 20
<210> 40
   <211> 20
   <212> DNA
   <213> Artificial sequence
<220>
   <223> Synthetic DNA
<400> 40
   gactcgtttc gcgatccgat 20
<210> 41
   <211> 20
   <212> DNA
   <213> Artificial sequence
<220>
   <223> synthetic DNA
<400> 41
   ctctggacac atggcagatc 20
<210> 42
   <211> 20
   <212> DNA
   <213> Artificial sequence
<220>
   <223> synthetic DNA
<400> 42
   ctctggaacc agtgcagatc 20

## Description

### BACKGROUND OF THE INVENTION

### Field of the Invention

The present invention relates to methods for improving cold acclimatation and freezing tolerance in plants.

### Discussion of the Background

Cold is an environmental factor that limits the geographical distribution and growing season of many plant species, and it often adversely affects crop quality and productivity (Thomashow 1999). For example, most temperate plants can acquire tolerance to freezing temperatures by a process known as cold acclimation in which tolerance arises through a prior exposure to low non-freezing temperatures (Guy 1990; Hughes and Dunn 1996; Browse and Xin 2001). However, plants of tropical and sub-tropical origins are incapable of cold acclimation and, as such, are sensitive to chilling temperatures (0-10°C). Many studies have suggested that cold-regulated gene expression is critical in plants for both chilling tolerance (Gong et al. 2002; Hsieh et al. 2002) and cold acclimation (Thomashow 1999; Knight et al. 1999; Tahtiharju and Palva 2001). Cold-responsive genes encode a diverse array of proteins, such as enzymes involved in respiration and metabolism of carbohydrates, lipids, phenylpropanoids and antioxidants; molecular chaperones, antifreeze proteins; and others with a presumed function in tolerance to the dehydration caused by freezing (Thomashow 1999; Guy 1990; Mohapatra et al. 1989).

Many of the cold and dehydration responsive genes have one or several copies of the *DRE*/*CRT* cis-element in their promoters, which has the core sequence, CCGAC (Yamaguchi-Shinozaki and Shinozaki 1994; Stockinger et al. 1997). A family of transcription factors known as CBFs or DREB1s binds to this element and activates transcription of the downstream cold and dehydration-responsive genes (Stockinger et al. 1997; Liu et al. 1998). Interestingly, the *CBF*/*DREB1* genes are themselves induced by low temperatures. This induction is transient and precedes that of the downstream genes with the *DRE*/*CRT cis*-element (Thomashow 1999). Therefore, there is a transcriptional cascade leading to the expression of the *DRE*/*CRT* class of genes under cold stress. Ectopic expression of *CBFs*/*DREBIs* in plants turns on downstream cold-responsive genes even at warm temperatures and confers improved freezing tolerance (Jagglo-Ottosen et al. 1998; Liu et al. 1998).

Since *CBF* transcripts begin accumulating within 15 min of plants' exposure to cold, Gilmour et al (1998) proposed that there is a transcription factor already present in the cell at normal growth temperature that recognizes the *CBF* promoters and induces *CBF* expression upon exposure to cold stress. Gilmour et al (1998) named the unknown activator(s) as "ICE" (Inducer of *CBF* Expression) protein(s) and hypothesized that upon exposing a plant to cold, modification of either ICE or an associated protein would allow ICE to bind to *CBF* promoters and activate *CBF* transcription.

Genetic analysis in *Arabidopsis* plants expressing the firefly luciferase reporter gene driven by the *CRT*/*DRE* element-containing *RD29A* promoter (Ishitani et al. 1997) has identified several mutants with de-regulated cold-responsive gene expression. The *hos1* (h*igh expression of* os*motically responsive genes)* mutant shows an enhanced cold-induction of *CBFs* and their downstream cold responsive genes (Ishitani et al. 1998). HOSI encodes a RING finger protein that is present in the cytoplasm at normal growth temperatures but accumulates in the nucleus upon cold treatment. Since many RING-finger proteins are known to serve as ubiquitin E3 ligases, HOS 1 has been proposed to function by targeting certain positive regulator(s) of *CBFs* for ubiquitination and degradation (Lee et al. 2001). The transcription of *CBF* genes is also under feedback repression by its own gene product or its downstream target gene products. This was revealed by studies on the *los1* mutant that is defective in the translational elongation factor 2 gene (Guo et al. 2002). The *Ios1* mutation blocks cold induction of genes with the *CRT*/*DRE* element but causes super-induction of the *CBF* genes. It was shown that protein synthesis in *los1* mutant plants is disrupted specifically in the cold. Therefore, cold-induced *CBF* transcripts cannot be translated to activate downstream genes, and feedback repression cannot occur, leading to super-induction of *CBF* transcripts (Guo et al. 2002).

Another *Arabidopsis* mutation, *los2,* also impairs cold induction of *CRT*/*DRE* element-containing genes (Lee et al., 2002). *LOS2* encodes a bi-functional enolase that can bind to the promoter of *ZAT10,* a zinc finger transcriptional repressor. *ZAT10* expression is rapidly and transiently induced by cold in the wild type, and this induction is stronger and more sustained in the *los2* mutant. Therefore, LOS2 may control the expression of delayed cold response genes via transcriptional repression of *ZAT10* (Lee et al. 2002). The *Arabidopsis LOS4* locus is involved in the accumulation of *CBF* transcripts under cold treatment (Gong et al. 2002). *los4-1* mutant plants are sensitive to chilling stress, and the chilling sensitivity can be rescued by ectopic expression of *CBF3* (Gong et al. 2002). *LOS4* encodes a DEAD-box RNA helicase, suggesting that RNA metabolism may be involved in cold responses.

Since environmental factors, such as cold, limits the geographical distribution and growing season of many plant species, and often adversely affects crop quality and productivity, there remains an ongoing critical need to increase cold acclimation in plants, particularly those plants that are advantageously useful as agricultural crops.

### SUMMARY OF THE INVENTION

It is an object of the present invention to provide methods for increasing cold acclimation in plants.

It is another object of the present invention to provide plants and plant cells, which have increased cold acclimation.

The objects of the present invention, and others, may be accomplished with a method of increasing cold acclimation in a plant, comprising overexpressing ICE1 in the plant.

The objects of the present invention may also be accomplished with a method of increasing cold acclimation in a plant cell, comprising overexpressing ICE1 in the plant cell.

The objects of the present invention may also be accomplished with a plant or a plant cell transformed with a nucleic acid that encodes ICE1.

In the framework of the present invention may be used: a method of producing such a plant or plant cell, by transforming a plant or plant cell with the nucleic acid that encodes ICE1;
an isolated and purified ICE1 having the amino acid sequence of SEQ ID NO:2;
a method of producing the ICE1 described above, comprising culturing host cells that have been transformed with a nucleic acid encoding ICE1 under conditions in which ICE1 is expressed, and isolating ICE1;
an isolated and purified enzyme having ICE1 transcriptional activator activity, wherein the amino acid sequence of the enzyme has a homology of from 70% to less than 100% to SEQ ID NO: 2;
a method of producing the enzyme described above, comprising culturing host cells that have been transformed with a nucleic acid encoding the enzyme under conditions in which the enzyme is expressed, and isolating the enzyme.

The present invention also provides a method of increasing cold acclimation in a plant, comprising overexpressing an *ICE1* transcriptional activator in the plant.

The present invention also provides a method of increasing cold acclimation in a plant by increasing the expression of one or more additional transcription factors selected from the group consisting of a CBF transcription factor and a DREB1 transcription factor and/or by increasing expression of one or more cold-responsive genes.

The present invention has been accomplished using a genetic screen (Chinnusamy et al. 2002) to identify cold signaling components upstream of the CBF proteins. A cold-responsive bioluminescent *Arabidpsis* plant was engineered by expressing the firefly luciferase (*LUC*) coding sequence under the control of the *CBF3* promoter. Homozygous *CBF3-LUC* plants were chemically mutagenized and luminescence imaging isolated mutants with altered cold-induced *CBF3-LUC* expression. In the present specification, the Inventors report on the *ice1* (*for inducer of* C*BF* e*xpression 1*) mutant, which is impaired in the cold-induction of *CBF3-LUC* and is defective in cold acclimation. *ICE1* encodes a MYC-like basic helix-loop-helix transcriptional activator that binds to the *CBF3* promoter. Thus, *ICE1* plays a key role in regulating cold-responsive gene expression and cold tolerance in *Arabidopsis.*

The above objects highlight certain aspects of the invention. Additional objects, aspects and embodiments of the invention are found in the following detailed description of the invention.

### BRIEF DESCRIPTION OF THE FIGURES

A more complete appreciation of the invention and many of the attendant advantages thereof will be readily obtained as the same becomes better understood by reference to the following Figures in conjunction with the detailed description below.

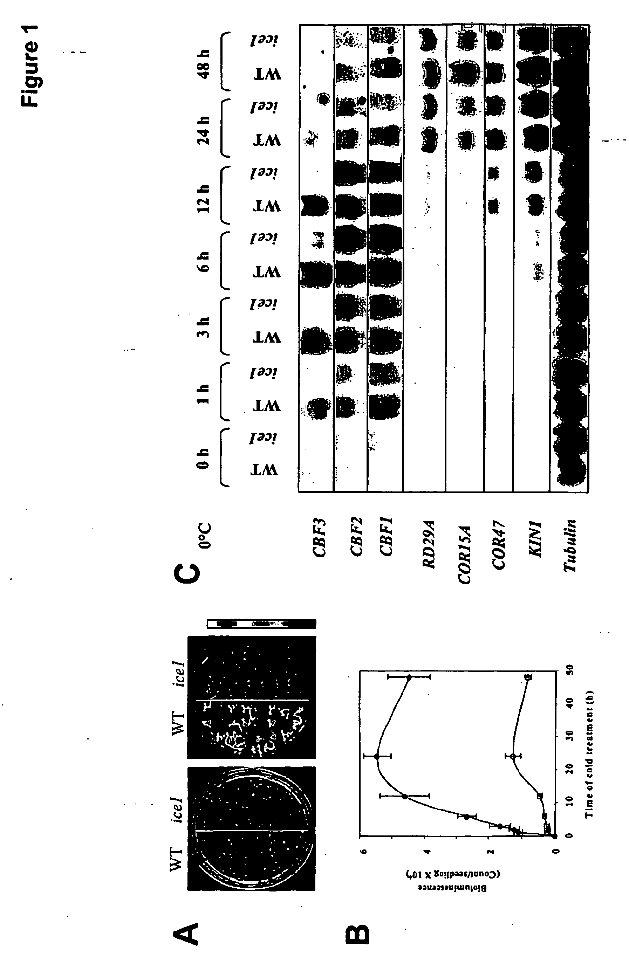

Figure 1. The *ice1* mutation blocks the cold-induction of CBF3 and affects the expression of other cold-responsive genes. (A) Morphology (left) and *CBF3-LUC* luminescence images (right) of wild-type and *icel* seedlings. Luminescence images of the plants were collected after 12 h of cold (0°C) treatment. (B) Quantitation of the luminescence intensities of wild type (solid circles) and *icel* (open circles) seedlings in response to different durations of cold treatment. (C) Transcript levels of *CBFs* and their downstream target genes in wild type and *ice1* plants in response to cold treatment. Seedlings were either not treated (0 h) or treated with cold (0°C) for the indicated durations (h). The tubulin gene was used as a loading control. WT, wild type.

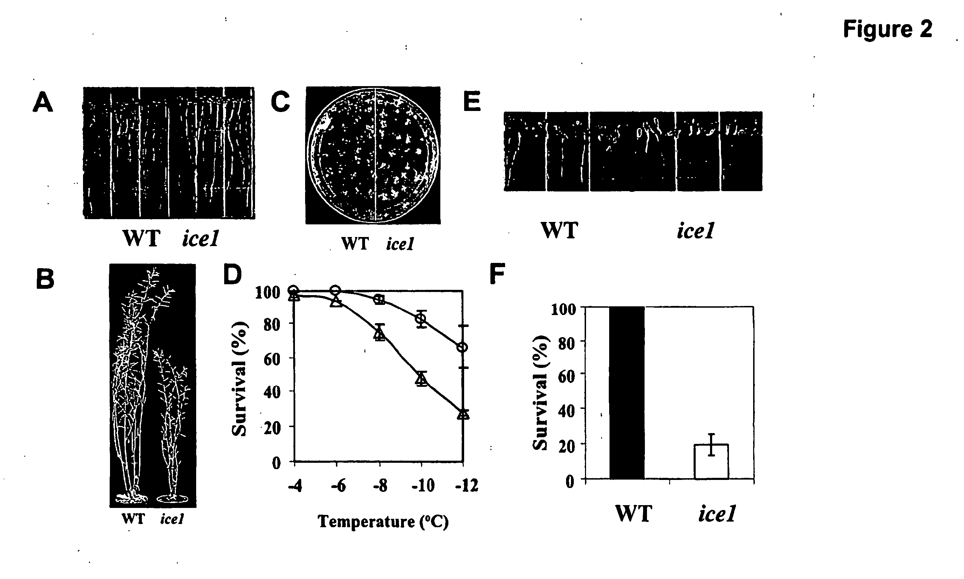

Figure 2. Morphology, and freezing and chilling sensitivity of *icel* mutant plants. (A) Wild type and *icel* seedlings in nutrient medium on agar under normal growth conditions. (B) Wild type and *ice1* plants in soil under normal growth conditions. (C) *icel* plants are defective in cold acclimation. Ten-day-old seedlings grown at 22°C were incubated for 4 days in light at 4°C before freezing treatment at -12°C. The picture was taken 3 days after the freezing treatment. (D) Comparison of survival rates after freezing treatments at the indicated temperatures. Open circles and open triangles represent wild type and *ice1* plants, respectively. (E) *ice1* plants are sensitive to prolonged chilling treatment. After germination at 22°C, the plants were grown at 4°C for 6 weeks. (F) Comparison of survival rates after 6 weeks of chilling stress.

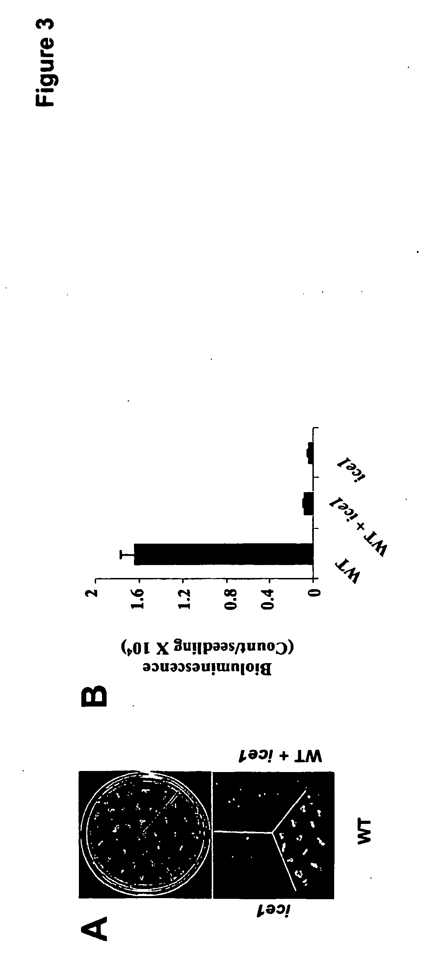

Figure 3. Confirmation of *ICE1* gene cloning by expressing the dominant *icel* mutant allele in wild type plants. (A) Expression in wild type of a genomic fragment containing the *ice1* mutation recapitulates the *ice1* mutant phenotype. Seven-day-old seedlings of the wild type, *ice1,* and wild type transformed with the mutant *ice1* gene grown on MS agar medium were subjected to luminescence imaging after 12 h of cold (0°C) stress. (B) Quantitation of *CBF3-LUC* bioluminescence levels in wild type (WT), *ice1* and WT transformed with the mutant *ice1* gene after 12 h of cold (0°C) stress.

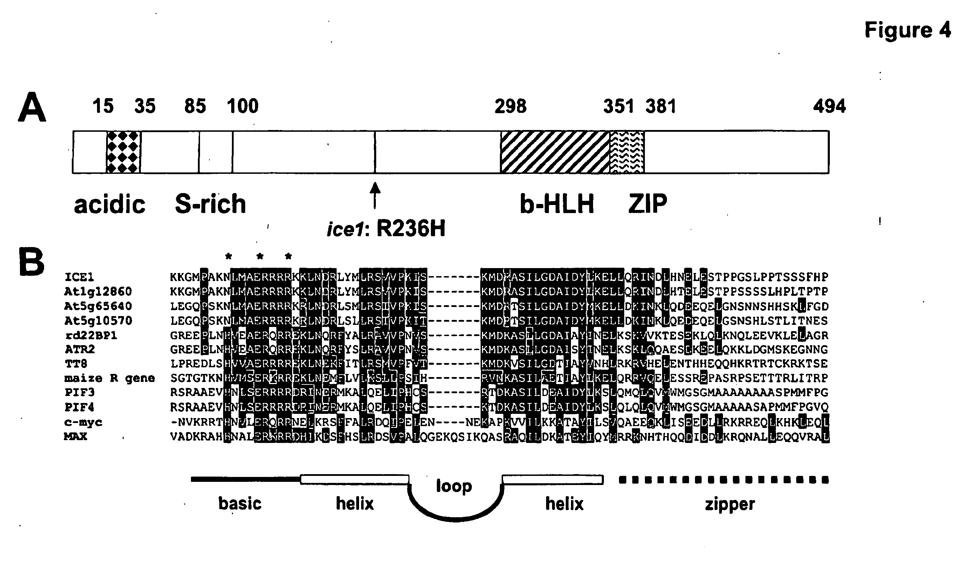

Figure 4. *ICE1* encodes a bHLH protein. (A) Overall domain structure of *ICE1* protein. A putative acidic domain (acidic), serine rich region (S-rich), bHLH domain, and possible zipper region (ZIP) are indicated. The arrow indicates the amino acid residue changed in the *ice1* mutant. (B) Sequence alignment of the bHLH domains and ZIP regions of *ICE1* and other plant and animal bHLH proteins. Identical and similar residues are shown in black and gray, respectively. A bold line indicates the basic region and open boxes connected with a loop indicate the helix-loop-helix domain. The zipper region is indicated as a dotted line. DDJB/EMBL/GenBank accession numbers and amino acid numbers (in parentheses) are: *ICE1* (SEQ ID NO: 2), AY195621 (300-398); At1g12860 (SEQ ID NO: 3), NM_101157 (638-731); At5g65640 (SEQ ID NO: 4), NM_125962.1 (171-269); At5g10570 (SEQ ID NO: 5), NM_121095.2 (144-242); rd22BP (SEQ ID NO: 6), AB000875 (446-544); ATR2 (SEQ ID NO: 7), NM_124046.1 (409-507); maize R gene (SEQ ID NO: 8), M26227 (410-508); TT8 (SEQ ID NO: 9), AJ277509 (357-455); PIF3 (SEQ ID NO: 10), AF100166 (254-352); PIF4 (SEQ ID NO: 11), AJ440755 (255-353); MAX (SEQ ID NO: 12), P52161 (21-107); c-myc (SEQ ID NO: 13), 1001205A (354-435). Asterisks indicate amino acid residues of MAX that are known to interact with nucleotides (Grandori et al. 2000).

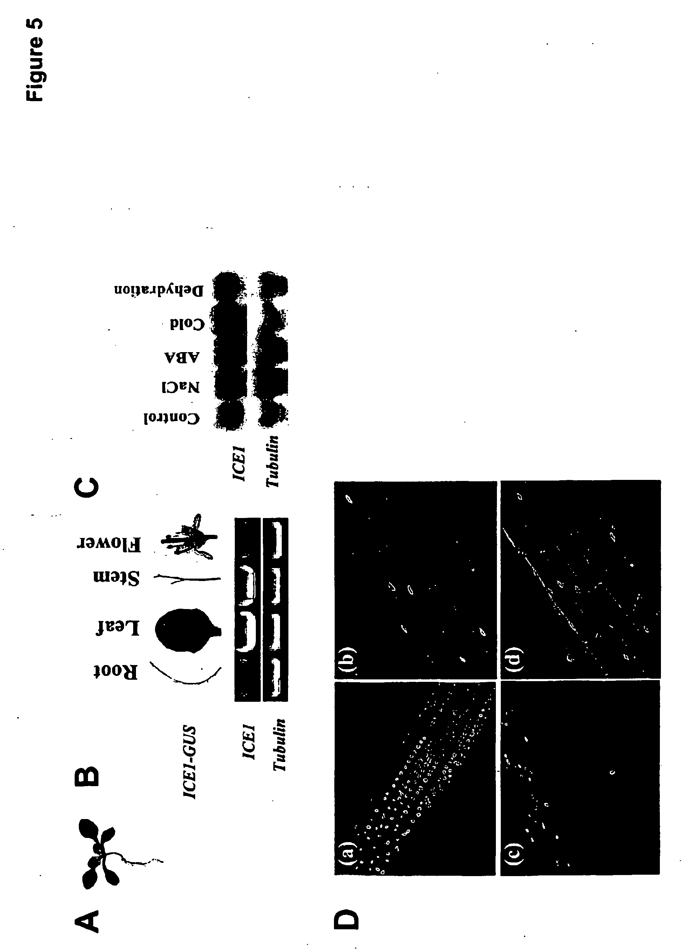

Figure 5. Expression of the *ICE1* gene and subcellular localization of the ICE1 protein. (A) *ICE1* promoter driven *GUS* expression pattern in a wild type seedling. (B) *ICE1* promoter-GUS expression in different plant tissues, and the corresponding *ICE1* transcript levels as determined by RT-PCR analysis. The tubulin gene was used as an internal control in the RT-PCR. (C) RNA blot analysis of *ICE1* expression in wild type seedlings under various abiotic stresses. Plants with the following treatments are shown: control, MS salt only; NaCl, 300 mM NaCl for 5 hr; ABA, 100 µM abscisic acid for 3 hr; Cold, 0°C for 2 hr; Dehydration, air drying for 30 min. (D) Localization of GFP-ICE1 fusion protein in the nucleus. Panels (a)-(c) show confocal images of root cells in *GFP-ICE1* transgenic plants, while panel (d) shows the location of nuclei as indicated by propidium stain.

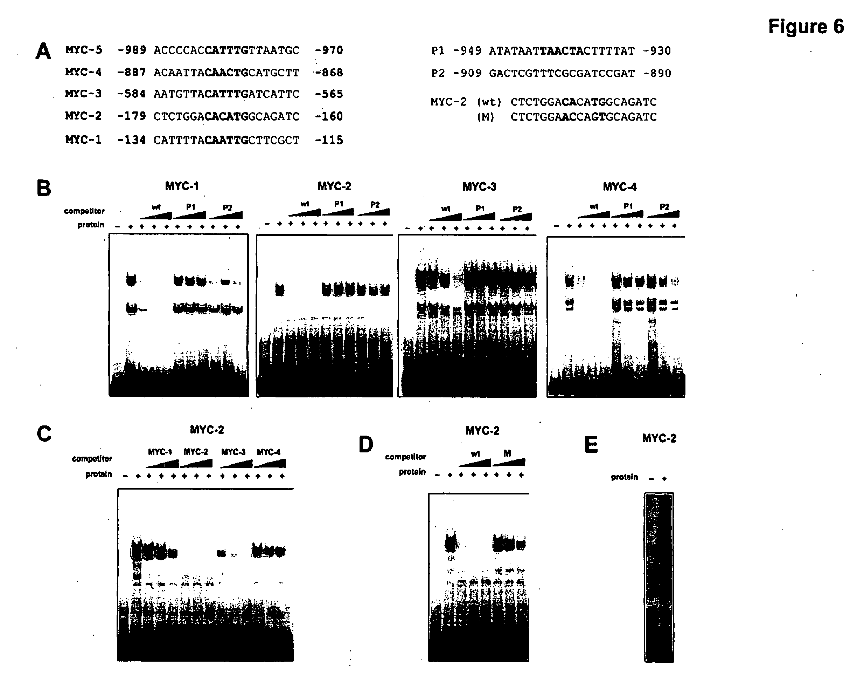

Figure 6. ICE1 protein binds to the MYC-recognition elements in the *CBF3* promoter. (A) Sequences and positions of oligonucleotides within the *CBF3* promoter used in the EMSA. Letters in bold indicate sequences of MYC-recognition motifs in MYC-1 (SEQ ID NO: 38), MYC-2 (SEQ ID NO: 37), MYC-3 (SEQ ID NO: 36), MYC-4 (SEQ ID NO: 35), and MYC-5 (SEQ ID NO: 34). Bold letters in the P1 (SEQ ID NO: 39) oligonucleotide are a putative MYB-recognition motif. The sequences labeled P2, MYC-2 (wt), and MYC-2 (M) correspond to (SEQ ID NO: 40), (SEQ ID NO: 41), and (SEQ ID NO: 42), respectively. (B) Interaction between ICE1 protein and ³²P-labeled MYC-1 through MYC-4 DNA fragments. (C) ICE1 binds to the MYC-2 DNA fragment more strongly than to the other DNA fragments. (D) Consensus nucleotide residues in the MYC-recognition motif are important for the interaction between ICE1 and the MYC-2 DNA fragment. (E) icel mutant protein also binds to the MYC-2 DNA fragment. The labeled oligonucleotides used in each experiment are indicated on the top of each panel. Triangles indicate increasing amounts of unlabeled oligonucleotides for competition in (B), (C) and (D), which correspond to 50-, 100-and 250-fold excess of each probe.

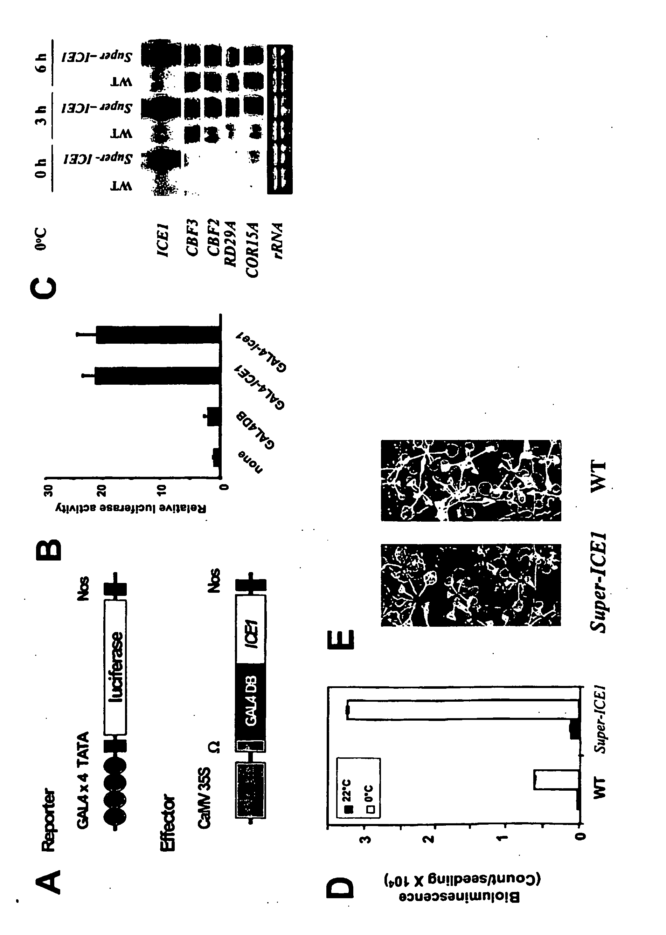

Figure 7. ICE1 is a transcriptional activator and its overexpression enhances the *CBF* regulon in the cold and improves freezing tolerance. (A) Schematic representation of the reporter and effector plasmids used in the transient expression assay. A *GAL4*-responsive reporter gene was used in this experiment. Nos denotes the terminator signal of the nopaline synthase gene. Ω indicates the translational enhancer of tobacco mosaic virus. GAL4 DB is the DNA binding domain of the yeast transcription factor GAL4. (B) Relative luciferase activities after transfection with *GAL4-LUC* and *35S-GAL4-ICE1* or *35S-GAL4-ice1.* To normalize values obtained after each transfection, a gene for luciferase from *Renilla* was used as an internal control. Luciferase activity is expressed in arbitrary units relative to the activity of *Renilla* luciferase (as described in Ohta et al. 2001). The values are averages of three bombardments, and error bars indicate standard deviations. (C) RNA blot analysis of *ICE1* and cold responsive gene expression in wild type and *ICE1* overexpressing transgenic (*Super-ICE1*) plants. Seedlings were either not treated (0 h) or treated with low temperature (0°C) for 3 h or 6 h. Ethidium bromide stained rRNA bands are shown as loading control. (D) *CBF3-LUC* expression (indicated as luminescence intensity) in wild type and *ICE1* overexpressing transgenic (*Super-ICE1*) plants. (E) Improved survival of *ICE1* overexpressing transgenic (*Super-ICE1*) plants after a freezing treatment.

### DETA1LED DESCRIPTION OF THE INVENTION

Unless specifically defined, all technical and scientific terms used herein have the same meaning as commonly understood by a skilled artisan in biochemistry, cellular biology, and molecular biology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will control. Further, the materials, methods, and examples are illustrative only and are not intended to be limiting, unless otherwise specified.

Reference is made to standard textbooks of molecular biology that contain definitions and methods and means for carrying out basic techniques, encompassed by the present invention. See, for example, Maniatis et al., Molecular Cloning: A Laboratory Manual, Cold Spring Harbor Laboratory Press, New York (1982) and Sambrook et al., Molecular Cloning: A Laboratory Manual, Cold Spring Harbor Laboratory Press, New York (1989); Methods in Plant Molecular Biology, Maliga et al, Eds., Cold Spring Harbor Laboratory Press, New York (1995); Arabidopsis, Meyerowitz et al, Eds., Cold Spring Harbor Laboratory Press, New York (1994) and the various references cited therein.

The term "plant" includes whole plants, plant organs (e.g., leaves, stems, roots, etc.), seeds and plant cells and progeny of same. The class of plants, which can be used in the methods of the invention, is generally as broad as the class of higher plants amenable to transformation techniques, including both monocotyledonous and dicotyledonous plants. Preferred plants include rice, corn, wheat, cotton, peanut, and soybean.

Thus, in one embodiment of the present invention, cold acclimation can be enhanced or increased by increasing the amount of protein available in the plant, preferably by the enhancement of the *ice1* gene in the plant.

Thus, one embodiment of the present invention uses plant cells carrying the polynucleotides useful in the present invention, and preferably transgenic plants carrying the isolated polynucleotides useful in the present invention.

As used herein, the term "enhancement" means increasing the intracellular activity of one or more enzymes in a plant cell and/or plant, which are encoded by the corresponding DNA. Enhancement can be achieved with the aid of various manipulations of the bacterial cell. In order to achieve enhancement, particularly over-expression, the number of copies of the corresponding gene can be increased, a strong promoter can be used, or the promoter- and regulation region or the ribosome binding site which is situated upstream of the structural gene can be mutated. Expression cassettes which are incorporated upstream of the structural gene may act in the same manner. In addition, it is possible to increase expression by employing inducible promoters. A gene can also be used which encodes a corresponding enzyme with a high activity. Expression can also be improved by measures for extending the life of the mRNA. Furthermore, preventing the degradation of the enzyme increases enzyme activity as a whole. Moreover, these measures can optionally be combined in any desired manner. These and other methods for altering gene activity in a plant are known as described, for example, in Methods in Plant Molecular Biology, Maliga et al, Eds., Cold Spring Harbor Laboratory Press, New York (1995).

An "expression cassette" as used herein includes a promoter, which is functional in a plant cell, operably linked to an isolated nucleic acid encoding an ICE1 protein of SEQ ID NO: 2, wherein enhanced expression of the protein in a plant cell imparts increased cold acclimation to said plant cell. In a preferred embodiment of the present invention the promoter is selected from the group consisting of a viral coat protein promoter, a tissue-specific promoter, a monocot promoter, a ubiquitin promoter, a stress inducible promoter, a CaMV 35S promoter, a CaMV 19S promoter, an actin promoter, a cab promoter, a sucrose synthase promoter, a tubulin promoter, a napin R gene complex promoter, a tomato E8 promoter, a patatin promoter, a mannopine synthase promoter, a soybean seed protein glycinin promoter, a soybean vegetative storage protein promoter, a bacteriophage SP6 promoter, a bacteriophage T3 promoter, a bacteriophage T7 promoter, a Ptac promoter, a root-cell promoter, an ABA-inducible promoter and a turgor-inducible promoter.

A gene can also be used which encodes a corresponding or variant enzyme with a high activity. Preferably the corresponding enzyme has a greater activity than the native form of the enzyme, more preferably at least in the range of 5, 10, 25% or 50% more activity, most preferably more than twice the activity of the native enzyme.

In the context of the present Application, a polynucleotide sequence is "homologous" with the sequence according to the invention if at least 70%, preferably at least 80%, most preferably at least 90% of its base composition and base sequence corresponds to the sequence according to the invention. According to the invention, a "homologous protein" is to be understood to comprise proteins which contain an amino acid sequence at least 70 % of which, preferably at least 80 % of which, most preferably at least 90 % of which, corresponds to the amino acid sequence which is shown in SEQ ID NO: 2 or which is encoded by the *ice1* gene (SEQ ID No. 1), wherein corresponds is to be understood to mean that the corresponding amino acids are either identical or are mutually homologous amino acids. The expression "homologous amino acids" denotes those that have corresponding properties, particularly with regard to their charge, hydrophobic character, steric properties, etc. Thus, the protein may be from 70% up to less than 100 % homologous to SEQ ID NO: 2.

Homology, sequence similarity or sequence identity of nucleotide or amino acid sequences may be determined conventionally by using known software or computer programs such as the *BesiFit* or *Gap* pairwise comparison programs (GCG Wisconsin Package, Genetics Computer Group, 575 Science Drive, Madison, Wisconsin 53711). *BestFit* uses the local homology algorithm of Smith and Waterman, Advances in Applied Mathematics 2: 482-489 (1981), to find the best segment of identity or similarity between two sequences. *Gap* performs global alignments: all of one sequence with all of another similar sequence using the method of Needleman and Wunsch, J. Mol. Biol. 48:443-453 (1970). When using a sequence alignment program such as *BestFit,* to determine the degree of sequence homology, similarity or identity, the default setting may be used, or an appropriate scoring matrix may be selected to optimize identity, similarity or homology scores. Similarly, when using a program such as *BestFit* to determine sequence identity, similarity or homology between two different amino acid sequences, the default settings may be used, or an appropriate scoring matrix, such as *blosum45* or *blosum80,* may be selected to optimize identity, similarity or homology scores.

The present invention also uses polynucleotides which contain the complete gene with the polynucleotide sequence corresponding to SEQ ID NO: I or fragments thereof, and which can be obtained by screening by means of the hybridization of a corresponding gene bank with a probe which contains the sequence of said polynucleotide corresponding to SEQ ID NO: I or a fragment thereof, and isolation of said DNA sequence.

Polynucleotide sequences useful in the invention are suitable as hybridization probes for RNA, cDNA and DNA, in order to isolate those cDNAs or genes which exhibit a high degree of similarity to the sequence of the *ice1* gene, in particular the *ice1* gene of SEQ ID NO: 1.

Polynucleotide sequences useful in the invention are also suitable as primers for polymerase chain reaction (PCR) for the production of DNA which encodes an enzyme having *ICE1* transcriptional activator activity.

Oligonucleotides such as these, which serve as probes or primers, can contain more than 30, preferably up to 30, more preferably up to 20, most preferably at least 15 successive nucleotides. Oligonucleotides with a length of at least 40 or 50 nucleotides are also suitable.

The term "isolated" means separated from its natural environment.

The term "polynucleotide" refers in general to polyribonucleotides and polydeoxyribonucleotides, and can denote an unmodified RNA or DNA or a modified RNA or DNA.

The term "polypeptides" is to be understood to mean peptides or proteins, which contain two or more amino acids which are bound via peptide bonds.

The polypeptides useful in the invention include polypeptides corresponding to SEQ ID NO: 2, particularly those with the biological activity of a *ICE1* transcriptional activator, and also includes those, at least 70 % of which, preferably at least 80% of which, are homologous with the polypeptide corresponding to SEQ ID NO: 2, and most preferably those which exhibit a homology of least 90 % to 95 % with the polypeptide corresponding to SEQ ID NO: 2 and which have the cited activity. Thus, the polypeptides may have a homology of from 70% up to 100% with respect to SEQ ID NO: 2.

The invention also uses coding DNA sequences, which result from SEQ ID NO: 1 by degeneration of the genetic code. In the same manner, the invention further uses DNA sequences which hybridize with SEQ ID NO: 1 or with parts of SEQ ID NO: 1. Moreover, one skilled in the art is also aware of conservative amino acid replacements such as the replacement of glycine by alanine or of aspartic acid by glutamic acid in proteins as "sense mutations" which do not result in any fundamental change in the activity of the protein, i.e. which are functionally neutral. It is also known that changes at the N- and/or C-terminus of a protein do not substantially impair the function thereof, and may even stabilize said function.

In the same manner, the present invention also uses DNA sequences which hybridize with SEQ ID NO: 1 or with parts of SEQ ID NO: 1. Finally, the present invention relates to DNA sequences which are produced by polymerase chain reaction (PCR) using oligonucleotide primers which result from SEQ ID NO: 1. Oligonucleotides of this type typically have a length of at least 15 nucleotides.

The terms "stringent conditions" or "stringent hybridization conditions" includes reference to conditions under which a polynucleotide will hybridize to its target sequence, to a detectably greater degree than other sequences (e.g., at least 2-fold over background). Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing).

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C., and a wash in 1X to 2X SSC (20X SSC=3.0 M NaCl/0.3 M trisodium citrate) at 50 to 55°C. Exemplary moderate stringency conditions include hybridization in 40 to 45% formamide, 1 M NaCl, 1% SDS at 37°C., and a wash in 0.5X to 1X SSC at 55 to 60°C. Exemplary high stringency conditions include hybridization in 50% formamide, 1 M NaCl, 1% SDS at 37°C., and a wash in 0.1X SSC at 60 to 65°C.

Specificity is typically the function of post-hybridization washes, the critical factors being the ionic strength and temperature of the final wash solution. For DNA-DNA hybrids, the Tm can be approximated from the equation of Meinkoth and Wahl, Anal. Biochem., 138:267-284 (1984): Tm =81.5°C.+16.6 (log M)+0.41 (%GC)-0.61 (% form)-500/L; where M is the molarity of monovalent cations, %GC is the percentage of guanosine and cytosine nucleotides in the DNA, % form is the percentage of formamide in the hybridization solution, and L is the length of the hybrid in base pairs. The Tm is the temperature (under defined ionic strength and pH) at which 50% of a complementary target sequence hybridizes to a perfectly matched probe. Tm is reduced by about 1°C for each 1% of mismatching; thus, Tm, hybridization and/or wash conditions can be adjusted to hybridize to sequences of the desired identity. For example, if sequences with approximately 90% identity are sought, the Tm can be decreased 10°C. Generally, stringent conditions are selected to be about 5°C lower than the thermal melting point (Tm) for the specific sequence and its complement at a defined ionic strength and pH. However, severely stringent conditions can utilize hybridization and/or wash at 1, 2, 3, or 4°C. lower than the thermal melting point (Tm); moderately stringent conditions can utilize a hybridization and/or wash at 6, 7, 8, 9, or 10°C lower than the thermal melting point (Tm); low stringency conditions can utilize a hybridization and/or wash at 11, 12, 13, 14, 15, or 20 °C lower than the thermal melting point (Tm). Using the equation, hybridization and wash compositions, and desired Tm, those of or ordinary skill will understand that variations in the stringency of hybridization and/or wash solutions are inherently described. If the desired degree of mismatching results in a Tm of less than 45°C (aqueous solution) or 32 °C (formamide solution) it is preferred to increase the SSC concentration so that a higher temperature can be used. An extensive guide to the hybridization of nucleic acids is found in Current Protocols in Molecular Biology, Chapter 2, Ausubel, et al., Eds., Greene Publishing and Wiley-Interscience, New York (2000).

Thus, with the foregoing information, the skilled artisan can identify and isolated polynucleotides that are substantially similar to the present polynucleotides. In so isolating such a polynucleotide, the polynucleotide can be used as the present polynucleotide in, for example, increasing cold acclimation of a plant.

One embodiment of the present invention is methods of screening for polynucleotides that have substantial homology to the polynucleotides of the present invention, preferably those polynucleotides encoding a protein having *ICEI* transcriptional activator activity.

The polynucleotide sequences useful in the present invention can be carried on one or more suitable plasmid vectors, as known in the art for plants or the like.

In one embodiment, it may be advantageous for propagating the polynucleotide to carry it in a bacterial or fungal strain with the appropriate vector suitable for the cell type. Common methods of propagating polynucleotides and producing proteins in these cell types are known in the art and are described, for example, in Maniatis et al., Molecular Cloning: A Laboratory Manual, Cold Spring Harbor Laboratory Press, New York (1982) and Sambrook et al., Molecular Cloning: A Laboratory Manual, Cold Spring Harbor Laboratory Press, New York (1989).

In another preferred embodiment the polynucleotide comprises SEQ ID NO: 1, polynucleotides which are complimentary to SEQ ID NO: 1, polynucleotides which are at least 70%, 80% and 90% identical to SEQ ID NO: 1; or those sequence which hybridize under stringent conditions to SEQ ID NO: 1, the stringent conditions comprise washing in 5X SSC at a temperature from 50 to 68°C. Thus, the polynucleotide may be from 70% up to less than 100% identical to SEQ ID NO: 1.

In another preferred embodiment the polynucleotides useful in the present invention are in a vector and/or a host cell. Preferably, the polynucleotides are in a plant cell or transgenic plant. Preferably, the plant is *Arabidopsis thaliania* or selected from the group consisting of wheat, corn, peanut cotton, oat, and soybean plant. In a preferred embodiment, the polynucleotides are operably linked to a promoter, preferably an inducible prompter.

In the framework of the present invention may be used: a process for screening for polynucleotides which encode a protein having *ICE1* transcriptional activator activity comprising hybridizing the polynucleotide of the invention to the polynucleotide to be screened; expressing the polynucleotide to produce a protein; and detecting the presence or absence of *ICE1* transcriptional activator activity in the protein;
a method for detecting a nucleic acid with at least 70% homology to nucleotide SEQ ID NO: 1, sequences which are complimentary to SEQ ID NO: 1 and/or which encode a protein having the amino acid sequence in SEQ ID NO: 2 comprising contacting a nucleic acid sample with a probe or primer comprising at least 15 consecutive nucleotides of the nucleotide sequence of SEQ ID NO: 1, or at least 15 consecutive nucleotides of the complement thereof;
a method for producing a nucleic acid with at least 70% homology to the polynucleotides of the present invention comprising contacting a nucleic acid sample with a primer comprising at least 15 consecutive nucleotides of the nucleotide sequence of SEQ ID NO: 1, or at least 15 consecutive nucleotides of the complement thereof;
a method for making *ICE1* protein, comprising culturing the host cell carrying the polynucleotides of the invention for a time and under conditions suitable for expression of ICE1, and collecting the ICE1;
a method of making a transgenic plant comprising introducing the polynucleotides of the invention into the plant. In another preferred embodiment, the present invention provides method of increasing cold acclimation of a plant in need thereof, comprising introducing the polynucleotides of the invention into said plant.

Methods, vectors, and compositions for transforming plants and plant cells useful in the invention are well-known to those skilled in the art, and are not particularly limited. For a descriptive example see Karimi et al., TRENDS in Plant Science, Vol. 7, NO: 5, May 2002, pp. 193-195, incorporated herein by reference.

In another preferred embodiment, the present invention provides an isolated polypeptide comprising the amino acid sequence in SEQ ID NO: 2 or those proteins that are at least 70%, preferably 80%, preferably 90% and preferably 95% identity to SEQ ID NO: 2, where the polypeptides have *ICE1* transcriptional activator activity. Thus, the enzyme has a homology of from 70% to less than 100% homology to SEQ ID NO: 2.

In another embodiment, the present invention also provides a method of increasing cold acclimation in a plant, comprising overexpressing an *ICE1* transcriptional activator in the plant.

The present invention also provides, in another embodiment a method of increasing cold acclimation in a plant by increasing the expression of one or more additional transcription factors selected from the group consisting of a CBF transcription factor and a DREB 1 transcription factor and/or by increasing expression of one or more cold-responsive genes.

In the context of the present invention the term "cold responsive genes" include genes that encode a protein selected from the group consisting of an enzyme involved in respiration of carbohydrates, an enzyme involved in metabolism of carbohydrates, an enzyme involved in respiration of lipids, an enzyme involved in metabolism of lipids, an enzyme involved in respiration of phenylpropanoids, an enzyme involved in metabolism of phenylpropanoids, an enzyme involved in respiration of antioxidants, an enzyme involved in metabolism of antioxidants, a molecular chaperone, an antifreeze protein, and a protein involved in tolerance to the dehydration caused by freezing.

The present invention has been accomplished using a genetic screen (Chinnusamy et al. 2002) to identify cold signaling components upstream of the CBF proteins. A cold-responsive bioluminescent *Arabidpsis* plant was engineered by expressing the firefly luciferase (*LUC*) coding sequence under the control of the *CBF3* promoter. Homozygous *CBF3-LUC* plants were chemically mutagenized and luminescence imaging isolated mutants with altered cold-induced *CBF3-LUC* expression. In the present specification, the Inventors report on the *ice1* (*for* i*nducer of* C*BF* e*xpression 1*) mutant, which is impaired in the cold-induction of *CBF3-LUC* and is defective in cold acclimation. *ICE1* encodes a MYC-like basic helix-loop-helix transcriptional activator that binds to the *CBF3* promoter. Thus, *ICE1* plays a key role in regulating cold-responsive gene expression and cold tolerance in *Arabidopsis.*

### Discussion

Cold temperatures trigger the transcription of the CBF family of transcription factors, which in turn activate the transcription of genes containing the *DRE*/*CRT* promoter element (Thomashow 1999). The CBF target genes presumably include some transcription factors (Fowler and Thomashow 2002). Therefore, cold signaling for freezing tolerance requires a cascade of transcriptional regulations. In the present study, we have identified ICE1, a very upstream transcription factor of this cascade. Our results show that ICE1 is a positive regulator of *CBF3* and has a critical role in cold acclimation. *ICE1* encodes a MYC-like bHLH transcription factor. Five putative MYC recognition sequences are present in the *CBF3* promoter, while *CBF1* and *CBF2* promoters each contain one such element (Shinwari et al. 1998). This is consistent with the fact that *CBF3* is more strongly affected by the *ice1* mutation than are *CBF1* or *CBF2.* DNA binding assays showed that ICE1 can specifically bind to the MYC recognition sequences on the *CBF3* promoter but not to a putative MYB recognition sequence (Fig. 6). The *ice1* mutation abolishes *CBF3* expression, and reduces the expression of CBF-target genes in the cold. Consistent with its role in cold-responsive gene regulation, ICE1 is important for chilling and freezing tolerance of *Arabidopsis* plants.

The *ice1* mutation also affects the cold-induction of *CBF1* and *CBF2;* their expression is slightly reduced early in the cold, but at later time points the expression is not reduced. Instead, the expression of *CBF2* is actually enhanced in the *ice1* mutant after 6 and 12 hours of cold treatment. The expression of *CBF* genes is known to be repressed by their gene products or the products of their downstream target genes (Guo et al. 2002). The correlation between the reduced *CBF3* expression and enhanced *CBF2* induction suggests that CBF3 may repress *CBF2* expression. When the *CBF2* gene is disrupted, *CBF1* and *CBF3* show more sustained induction in the cold (Julio Salinas, personal communication), suggesting that CBF2 may repress the expression of *CBF1* and *CBF3.* The potential negative regulation of each other among the CBF transcription factor genes may be important for ensuring that their expression is transient and tightly controlled.

The three *CBF* genes are generally presumed to be functionally redundant. Their individual contribution has not been examined by loss of function analysis. Even though the *icel* mutation only blocks the expression of *CBF3,* the downstream genes such as *RD29A, COR15A* and *COR47* are substantially affected. This suggests that CBF3 plays a critical role in the cold regulation of these genes. In comparison, the cold regulation of *KINI* is less affected by the *ice1* mutation. Therefore, it is possible that the three *CBF* genes may each have their own set of preferred target genes.

*ICE1* is expressed constitutively in all tissues (Figs. 5A and 5B), and is only slightly up-regulated by cold (Fig. 5C). Consistent with what has been speculated for "ICE" proteins (Gilmour et al. 1998), cold induced modification of the ICE1 protein or of a transcriptional co-factor appears to be necessary for ICE1 to activate the expression of *CBFs.* Our evidence supports this because *ICE1* is expressed constitutively and localized in the nucleus, but the *CBF* expression requires cold treatment; and transgenic lines constitutively overexpressing *ICE1* do not show *CBF3* expression at warm temperatures but have a higher level of *CBF3* expression at cold temperatures. The ability of transcription factors to activate gene transcription may be regulated by protein phosphorylation and dephosphorylation in the cytoplasm or in the nucleus (reviewed by Liu et al. 1999). The *ice1* mutation is very near potential serine phosphorylation residues (Ser243 and Ser245), and thus might affect the phosphorylation/dephosphorylation of ICE1.

It is known that MYC-related bHLH transcription factors require MYB co-transcription factors and/or WD-repeat containing factors for transcriptional activation of target genes (Spelt et al. 2000; Walker et al. 1999). The promoters of *CBFs* contain MYC as well as potential MYB recognition sequences (Shinwari et al. 1998), suggesting that a MYB-related transcription factor may also be involved in the cold induction of *CBFs.* The *ice1* mutation, which substitutes Arg236 with His, may interfere with hetero-oligomer formation between ICE1 and an ICE1-like protein or a MYB-related co-factor. Alternatively, the putative dominant negative effect of *ice1* could be a consequence of *ice1* interference with potential ICE1 homo-oligomer formation, protein stability, nuclear localization, or cold induced post-translational modification of *ICE1.*

Having generally described this invention, a further understanding can be obtained by reference to certain specific examples, which are provided herein for purposes of illustration only, and are not intended to be limiting unless otherwise specified.

### EXAMPLES

### Materials and methods

**Plant materials and mutant isolation:** The CBF3 promoter, a region from 1126 to 100 bp upstream of the initiation codon, was obtained by polymerase chain reaction (PCR) using the following primer pair: 5'-TCATGGATCCACCATTTGTTAATGCATGATGG-3' (SEQ ID NO: 14) and 5'-GCTCAAGCTTTCTGTTCTAGTTCAGG-3' (SEQ ID NO: 15). This promoter was placed in front of the firefly luciferase (LUC) coding sequence in a plant transformation vector (Ishitani et al. 1997). Arabidopsis thaliana ecotype Columbia (with the glabrous1 mutation) was transformed with Agrobacterium tumefaciens containing this CBF3-LUC construct by the floral dipping method. Plants homozygous for the CBF3-LUC transgene were selected from the second generation after transformation. One such plant with a single copy of the CBF3-LUC transgene was chosen for subsequent experiments (hereafter referred to as wild type). This wild type plant did not show any bioluminescence when grown under normal growth conditions, but emitted bioluminescence when cold stress was imposed. The CBF3-LUC plant seeds were mutagenized with ethyl methanesulfonate (EMS). Seedlings of the M2 generation were used to screen for mutants defective in cold regulated CBF3-LUC expression by luminescence imaging. Seven-day-old seedlings grown on 0.6% agar plates containing 3% sucrose and 1x Murashige and Skoog (MS) salts (JRH Biosciences) were screened for de-regulated luciferase expression in response to low temperature treatment at 0°C for 12 hours, using a low light video imaging system (Princeton Instruments). Luminescence intensities of individual seedlings were quantified with the WINVIEW software provided by the camera manufacturer (Princeton Instruments) (Chinnusamy et al. 2002).

**Chilling and freezing tolerance assays**: Chilling sensitivity of ice 1 and wild type plants were tested by exposing the seedlings immediately after radicle emergence. After 2 days of stratification at 4°C, mutant and wild-type seeds were germinated at 22°C on MS nutrient medium with 3% sucrose and 1.2% agar. Chilling stress was imposed by incubating the seedlings at 4 ± 1°C with 30 ± 2 µmol quanta. m⁻².s⁻¹ light. Freezing tolerance was assayed as described (Xin and Browse, 1998). Briefly, wild type and ice1 seeds were sown on agar (0.9%) plates with Gamborg basal salts and 1.5% sucrose. After 2 days of stratification at 4°C, the plates were kept at 22°C under 50 ± 2 µmol quanta m⁻².s⁻¹ continuous light. Ten-day-old seedlings were cold acclimated at 4 ± 1°C and 30 ± 2 µmol quanta. m⁻².s⁻¹ light for 4 days. These plants on petri dishes were placed on ice in a freezing chamber (Percival Scientific) set to -1 ± 0.1°C for 16 h. Ice chips were sprinkled on these plants before the chamber was programmed to cool at 1 °C h⁻¹. Petri dishes of plants were removed after being frozen at desired temperatures for 2 hours unless indicated otherwise, thawed at 4°C for 12 hours in the dark, and then transferred to 22°C under 50 ± 2 µmol quanta m⁻².s⁻¹ continuous light. Survival of the seedlings was scored visually after two days.

**Gene expression analysis:** For RNA analysis, ten-day-old seedlings of WT and icel plants grown on separate halves of the same MS agar plates were used. Total RNA extracted from control and stressed plants was analyzed by RNA blotting as described by Liu and Zhu (1997). The RD29A gene-specific probe was from the 3' noncoding region (Liu and Zhu 1997). COR15A and COR47 cDNAs (Gilmour et al. 1992; Lin and Thomashow 1992) were kindly provided by M. F. Thomashow (Michigan State University). The CBF2 and CBF3 gene-specific probes were generated by PCR with the following primer pairs: CBF2-forward primer, 5'-TTCGATTTTTATTTCCATTTTTGG-3' (SEQ ID NO: 16); CBF2-reverse primer, 5'-CCAAACGTCCTTGAGTCTTGAT-3' (SEQ ID NO: 17); CBF3-forward primer, 5'-TAAAACTCAGATTATTATTTCCATTT-3' (SEQ ID NO: 18); CBF3-reverse primer, 5'-GAGGAGCCACGTAGAGGGCC-3' (SEQ ID NO: 19). The probe for KIN1 (Kurkela and Franck, 1990) was a 0.4-kb EcoRI fragment of the Arabidopsis EST clone YAP368T7. The β-tubulin gene was used as a loading control and was amplified by PCR with the following primer pairs: forward primer (5'-CGTGGATCACAGCAATACAGAGCC-3' (SEQ ID NO: 20)) and reverse primer (5'-CCTCCTGCACTTCCACTTCGTCTTC-3' (SEQ ID NO: 21)).

For Affymetrix GeneChip array analysis, 20 µg of total RNA from the wild type and icel seedlings with or without cold treatment (6 hours under light) were extracted using the RNeasy Plant Mini Kit (Qiagen) and used to make biotin-labeled cRNA targets. The Affymetrix Arabidopsis ATH1 genome array GeneChips, which contain more than 22,500 probe sets representing approximately 24,000 genes, were used and hybridization, washing, and staining were carried out as directed in the manufacturer's manual. Microarray data were extracted from scanned GeneChip images and analyzed using Microarray Suite version 5.0.1 (Affymetrix).

**Mapping and cloning of the ICE1 locus:** Genetic analysis of F₁ and F₂ progenies of the icel cross with WT showed that ice1 is a dominant mutation. Hence to clone ICE1, a homozygous ice1 plant was crossed with the Arabidopsis Landsberg erecta (Ler) ecotype and the F₂ progeny from self-pollinated F₁ were used to select mapping samples with the wild type phenotype. Genomic DNA extracted from these seedlings was used for PCR-based mapping with simple sequence polymorphism markers or cleaved amplified polymorphic sequence markers. New SSLP mapping markers on F16J4, MTC11, MLJ15, MDJ14, K17E12 and T32N15 BAC clones were developed based on insertion/deletions identified from the Cereon Arabidopsis polymorphism and Ler sequence collection (http://www.arabidopsis.org). Genomic DNA corresponding to candidate genes was amplified by PCR from icel mutant and wild type plants and sequenced to identify the icel mutation.

For ice1 mutant complementation, the MLJ15.14 gene, including 2,583 bp upstream of the initiation codon and 615 bp downstream of the stop codon, was PCR amplified by LA Taq polymerase (Takara) using ice1 mutant genomic DNA as template. The PCR primers used were as follows: forward primer: 5'-AGGGATCCGGACCACCGTCAATAACATCGTTAAGTAG-3' (SEQ ID NO: 22); reverse primer: 5'-CGAATTCTAACCGCCATAACTATGTCTCCTCTCTATCTC-3' (SEQ ID NO: 23). The resulting 5,035-bp fragment was T-A cloned into the pCR2.1 TOPO vector (Invitrogen) and then subcloned into pCAMBIA1200 between the BamHI and EcoRI sites. This and all other constructs described here were completely sequenced to ensure that they did not contain PCR or cloning errors. The binary construct was then introduced into Agrobacterium strain GV3101 and transformed into CBF3-LUC Columbia wild type plants. Hygromycin-resistant transgenic plants were selected and their T2 progenies were tested for CBF3-LUC expression in response to cold stress.

**Analysis of ICE1 expression:** The promoter region (2,589 bp upstream from the initiation codon) of the ICE1 gene was PCR-amplified with the following primer pair: forward primer, 5'-AGGGATCCGGACCACCGTCAATAACATCGTTAAGTAG-3' (SEQ ID NO: 24); reverse primer, 5'-CGAATTCGCCAAAGTTGACACCTTTACCCCAAAG-3' (SEQ ID NO: 25). The resulting fragment was digested with BamHI and EcoRI and inserted into the pCAMBIA1391 binary vector. This ICE1 promoter-GUS construct was introduced into Agrobacterium strain GV3101 and transformed into wild type Arabidopsis. T2 transgenic lines resistant to hygromycin were analyzed for ICE1-promoter driven GUS expression. For GUS staining, T2 seedlings grown on MS agar plates were incubated with X-Gluc. for 12 h at 37°C and then washed 5 times with 70% (v/v) ethanol at 70°C to remove chlorophyll. ICE1 expression was also examined by quantitative RT-PCR analysis of RNA prepared from wild type roots, leaves, stems and flowers. The ICE 1 cDNA was amplified by RT-PCR using the following primers: forward primer: 5'-GCGATGGGTCTTGACGGAAACAATGGTG-3' (SEQ ID NO: 26) and reverse primer 5'-TCAGATCATACCAGCATACCCTGCTGTATCG-3' (SEQ ID NO: 27). The tubulin gene was used as an internal control in the RT-PCR analysis. Tubulin cDNA was amplified using the following primers: forward primer: 5'-GTCAAGAGGTTCTCAGCAGTA-3' (SEQ ID NO: 28) and reverse primer 5'-TCACCTTCTTGATCCGCAGTT-3' (SEQ ID NO: 29).

**Overexpression of ICE1:** The ICE1 cDNA was amplified from Arabidopsis (ecotype Columbia) RNA by RT-PCR using the following primers: a forward primer: 5'-GCTCTAGAGCGATGGGTCTTGACGGAAACAATGGTG-3' (SEQ ID NO: 30) and a reverse primer 5'-GGGGTACCTCAGATCATACCAGCATACCCTGCTGTATCG-3' (SEQ ID NO: 31). The PCR product was digested with XbaI and KpnI, and cloned into the pBIB vector under control of the superpromoter, which consists of three copies of the octopine synthase upstream-activating sequence in front of the manopine synthase promoter (Li et al. 2001). Agrobacterium tumefaciens strain GV3101 containing this binary construct was used to transform Arabidopsis plants. Transformants were selected on MS medium containing hygromycin (30 mg/l).

**Expression and localization of GFP-ICE1 fusion protein:** The full-length ICE1 cDNA was obtained from wild type plants by RT-PCR using the following primers: forward primer, 5'-AGGAATTCGCGATGGGTCTTGACGGAAACAATGGTG-3' (SEQ ID NO: 32); reverse primer, 5'-CTGGATCCTCAGATCATACCAGCATACCCTGCTGTATCG-3' (SEQ ID NO: 33). The resulting PCR fragment was digested with EcoRI and BamHI and cloned into the binary vector pEGAD downstream from the CaMV 35S promoter. This GFP-ICEI construct was introduced into Agrobacterium strain GV3101 and transformed into wild type Arabidopsis. T2 transgenic lines resistant to Basta (glufosinate) were selected and analyzed for GFP expression. To visualize the nucleus, root tissues were stained with propidium iodide (1 µg/mL). Green fluorescence (GFP expression) and red fluorescence (propidium iodide staining) analyses of transgenic plants were performed with a confocal laser-scanning microscope.

**DNA binding assay:** The wild type and mutant ICE1 cDNAs were amplified by RT-PCR and inserted into NdeI and BamHI sites in the expression vector pET14b (Novagen). Wild-type and mutant His-ICE1 fusion proteins were prepared from E.coli cells (BL21 DE3) according to the instruction manual of His-Bind Buffer Kit (Novagen). The electrophoresis mobility shift assay (EMSA) was carried out as described (Hao et al. 1998). The following double-stranded oligonucleotides listed in Fig. 6A (MYC-1, MYC-2, MYC-3, MYC-4 and MYC-5) were used as probes and competitors in EMSAs. Nucleotide sequences P1 (-949 to -930) and P2 (-909 to -890) were also used as competitors. P1 contains a putative MYB-recognition site. P2 does not contain any typical cis-elements. DNA probes were end-labeled with [γ-³²P]dCTP using the Klenow fragment and purified through a Sephadex G-50 column. The labeled probes (ca 0.02 pmol) were incubated for 20 min at room temperature with 2.3 µg of purified His-ICE1 fusion protein in 1X binding buffer (Hao et a.1998) supplemented with 20 pmol poly(dI-dC). The resulting DNA-protein complexes were resolved by electrophoresis on a 6% polyacrylamide gel in 0.5X TBE buffer and visualized by autoradiography. For competition experiments, unlabeled competitors were incubated with the His-ICE1 fusion protein on ice for 30 min prior to the addition of labeled probes.

**Transient expression assay:** The wild type (ICE1) and mutant (icel) cDNAs were amplified by RT-PCR, digested with SalI and inserted into SmaI and SalI sites of the plant expression vector 35S-GAL4 DB (Ohta et al. 2000). The plasmid DNA of the resulting effector, GAL4-ICE1, and a GAL4 responsive reporter, GAL4-LUC (Ohta et al. 2000) were delivered into Arabidopsis leaves using particle bombardment (Ohta et al. 2001).

### Experimental Example

### Identification of the ICE1 locus:

Using the genetic screen noted above, *Arabidopsis* plants containing the *CBF3-LUC* transgene emitted bioluminescence in response to cold stress (Figs. 1A and 1B). The homozygous *CBF3-LUC* plants (herein referred to as wild type) were mutagenized by ethylmethane sulfonate, and the resulting M2 population was screened for mutants with aberrant bioluminescence responses under cold stress using a low light imaging system (Chinnusamy et al. 2002). Several mutants showing abnormal cold regulation of *CBF3-LUC* expression were recovered. One of these mutant lines, designated as *ice1,* is virtually blocked in *CBF3-LUC* expression in the cold (Figs. 1A and 1B). In response to treatment at 0°C, wild type plants showed strong luminescence, while the *ice1* mutant showed very little induction of luminescence throughout the duration of cold treatment (Figs. 1A and 1B). After 12 hours of cold treatment, *ice1* plants showed nearly 10 times less luminescence than that of wild type plants, and are obviously defective in the cold regulation of *CBF3-LUC* expression (Fig. 1B).

The *ice1* mutant plant was crossed with *CBF3-LUC* wild type plants and the resulting F1 plants were examined for *CBF3-LUC* expression after 12 hours of cold treatment at 0°C. As determined by luminescence imaging, all F1 plants showed reduced cold-induced *CBF3-LUC* expression similar to that of *ice1.* An F2 population from the selfed F1 segregated in an approximately 3 to 1 ratio between mutant and wild type. These results show that *ice1* is a dominant mutation in a single nuclear gene.

### ice1 mutant plants are defective in cold-regulated gene expression:

RNA blot analysis was carried out to analyze the effect of *ice1* mutation on the transcript levels of endogenous *CBFs* and their target cold stress-responsive genes. Consistent with the imaging results, cold induction of the endogenous *CBF3* gene was greatly impaired (almost abolished) in *ice1* mutant plants (Fig. 1C). Wild type plants showed *CBF3* induction after 1 hour of cold stress and the expression peaked at 6 hour. In contrast, *CBF3* induction was almost abolished in *ice1* plants (Fig. 1C). While the *CBF1* induction level was lower in the *ice1* mutant was lower than that of wild type at 1 and 3 hours of cold stress, its induction level at 6 and 12 hours was similar to that in the wild type. The *CBF2* induction level was slightly lower in *icel* at 1 hour of cold treatment, whereas at 6 and 12 hours, the induction level was higher in the mutant (Fig. 1C). We also examined the cold induction of the downstream target genes of *CBFs.* The expression levels of *RD29A, COR15A* and *COR47A* under cold stress were lower in *icel* than in the wild type, while the induction of *KIN1* was lower in *ice1* only after 48 hours of cold stress (Fig. 1C).

Consistent with these RNA blot results, microarray analysis using Affymetrix near full genome genechips showed that out of 306 genes induced 3-fold or more in the wild type by a 6-hour cold treatment, 217 are either not induced in the *icel* mutant or their induction is 50% or less of that in the wild type (Table 1A). Thirty-two of these encode putative transcription factors, suggesting that ICE1 may control many cold-responsive regulons. For 87 of the 306 cold induced genes, their induction levels in the wild type and *ice1* differ by less than 2-fold (Table 1B). Interestingly, 2 genes show higher levels of cold induction in the *ice1* mutant (Table 1C).
Table 1. Cold-responsive gene expression in the wild type and *ice1.*

For cold treatment, the wild-type and *ice1* seedlings were placed at 0 ±1 °C under light for 6 hours. Affymetrix GeneChip analysis was carried out as described in materials and methods. Gene expression changes were analyzed by comparing values for a cold-treated sample to those for a control sample in each genotype. 'Fold Change' value of +1 or - 1 indicates no change in gene expression. Up-regulation or down-regulation is expressed by either + or - in 'Fold Change' values, respectively. Cold-responsive genes were determined in the wild type by the following standards; 1) signal intensities from cold treated sample were greater than background (i.e. genes with 'Present' calls, determined by Affymetrix Microarray Suite Program, in a cold treated sample); 2) 'Change' calls, made by Affymetrix Microarray Suite, in pair-comparison were 'I' (for 'increase'); 3) the 'Fold Change' in pair-comparison was 3-fold or higher. The expression of the resulting 306 genes was further analyzed and compared with that in *ice1* mutant. A two-fold difference between changes in the wild type and *ice1* was used as a threshold to categorize genes. Transcription factors are shown in gray blocks. Genes used for RNA hybridization analysis are in bold. The fold change values for 22 genes in cold-treated *ice1* were not determined (ND) because their signal intensities were similar to the background value (i.e. genes with 'Absent' calls in cold-treated *ice1*). These 22 genes were all cold-induced in the wild type. Therefore, they were included in the category of cold-responsive genes with lower induction in *ice1* than in the wild type.

**Table 1A: Cold-responsive genes with lower induction in ice1**

| Probe Set | AGI ID | Gene Title | Fold Change | |
|---|---|---|---|---|
| | | | WT | *icel* |
| 254074_at | At4g25490 | **CHF1/DREB1B** | 445.7 | 64.0 |
| 254066_at | At4g25480 | **CBF3/DREB1A** | 78.8 | 29.9 |
| 258325_at | At3g22830 | putative heat shock transcription factor1 | 42.2 | 5.7 |
| 246432_at | At5g17490 | RGA-like protein | 34.3 | ND |
| 261648_at | At1g27730 | salt-tolerance zinc finger protein | 24.3 | 6.5 |
| 247655_at | At5g59820 | zinc finger protein Zat12 | 19.7 | 7.0 |
| 248160_at | At5g54470 | CONSTANS B-box zinc finger family protein | 19.7 | 9.8 |
| 250781_at | At5g05410 | DRE binding protein (DREB2A) | 14.9 | 4.9 |
| 251745_at | At3g55980 | Zn ringer transcription factor (PEI1) | 13.9 | 3.0 |
| 258139_at | At3g24520 | heat shock transcription factor HSF1, putative | 13.9 | 3.7 |
| 245711_at | At5g04340 | putative c2h2 zinc finger transcription factor | 11.3 | 5.3 |
| 245250_at | At4g17490 | ethylene-responsive element binding factor 6 (AtERF6) | 8.6 | 4.3 |
| 252214_at | At3g50260 | EREBP-3 homolog | 8.6 | 2.1 |
| 261613_at | At1g49720 | abscisic acid responsive elements-binding factor | 7.0 | 3.5 |
| 245078_at | At2g23340 | putative AP2 domain transcription factor | 5.7 | 1.4 |
| 263379_at | At2g40140 | putative CCCH-type zinc finger protein | 5.7 | 2.6 |
| 253405_at | At4g32800 | transcription factor TINY, putative | 5.3 | ND |
| 245807_at | At1g46768 | AP2 domain protein RAP2.1 | 4.9 | 1.9 |
| 259432_at | At1g01520 | myb family transcription factor | 4.9 | 2.3 |
| 252278_at | At3g49530 | NAC2-like protein | 4.6 | 2.0 |
| 253485_at | At4g31800 | WRKY family transcription factor | 4.6 | -1.2 |
| 251272_at | At3g61890 | homeobox-lcucine zipper protein ATHB-12 | 4.3 | 1.3 |
| 261470_at | At1g28370 | cthylene-responsive clement binding factor 11 (AtERF11) | 4.3 | 1.7 |
| 261892_at | At1g80840 | WRKY family transcription factor | 4.3 | 1.2 |
| 263783_at | At2g46400 | WRKY family transcription factor | 4.3 | 1.4 |
| 257022_at | At3gt9580 | zinc finger protein, putative | 3.7 | 1.4 |
| 267252_at | At2g23100 | CHP-rich zinc finger protein, putative | 3.7 | ND |
| 249746_at | At5g24590 | NAC2-like protein | 3.5 | 1.6 |
| 256093_at | At1g20823 | putative RING zinc finger protein . | 3.5 | 1.4 |
| 252009_at | At3g52800 | zinc finger - like protein | 3.2 | 1.3 |
| 256185 at | At1g51700 | Dofzinc finger protein | 3.2 | 1.6 |
| 260763_at | At1g49220 | RING-H2 finger protein RHA3a, putative | 3.2 | ND |
| 245749_at | At1g51090 | proline-rich protein, putative | 73.5 | 7.5 |
| 264217_at | At1g60190 | hypothetical protein | 68.6 | 26.0 |
| 246467_at | At5g17040 | UDP glucose:flavonoid 3-o-glucosyltransferase -like protein | 29.9 | ND |
| 251793_at | At3g55580 | regulator of chromosome condensation-like protein | 27.9 | 6.1 |
| 262452_at | At1g11210 | expressed protein | 27.9 | 7.0 |
| 264661_at | At1g09950 | hypothetical protein | 27.9 | ND |
| 258947_at | At3g01830 | expressed protein | 26.0 | 2.5 |
| 246178_s_ at | At5g28430 | putative protein | 19.7 | 7.0 |
| 253104_at | At4g36010 | thaumatin-like protein | 19.7 | 2.5 |
| 257391_at | At2g32050 | hypothetical protein | 19.7 | ND |
| 245627_at | At1g56600 | water stress-induced protein, putative | 18.4 | ND |
| 247208_at | At5g64870 | nodulin-like | 18.4 | 1.2 |
| 256114_at | At1g16850 | expressed protein | 18.4 | 2.6 |
| 256356_s_at | At1g66500 | hypothetical protein | 18.4 | 3.0 |
| 250098_at | At5g17350 | putative protein | 17.1 | 3.2 |
| 264758_at | At1g61340 | late embryogenesis abundant protein, putative | 17.1 | 5.3 |
| 246099_at | At5g20230 | blue copper binding protein | 16.0 | 1.3 |
| 257280_at | At3g14440 | 9-cis-cpoxycarotenoid dioxygenasc (neoxanthin cleavage enzyme)(NC1)(NCED1), putative | 16.0 | 1.0 |
| 251336_at | At3g61190 | putative protein | 13.9 | 3.0 |
| 260264_at | At1g68500 | hypothetical protein | 13.9 | 3.0 |
| 263497_at | At2g42540 | COR15a | 13.9 | 3.5 |
| 248337_at | At5g52310 | RD29A/COR78/LT178 | 13.0 | 4.6 |
| 248959_at | At5g45630 | putative protein | 13.0 | 2.0 |
| 259977_at | At1g76590 | expressed protein | 13.0 | 2.5 |
| 260399_at | At1g72520 | putative lipoxygenase | 13.0 | 1.5 |
| 259879 at | At1g76650 | putative calmodulin | 12.1 | 2.8 |
| 265290_at | At2g22590 | putative anthocyanidin-3-glucoside rhamnosyltransferasc | 12.1 | ND |
| 267411 at | At2g34930 | disease resistance protein family | 12.1 | 1.1 |
| 250648_at | At5g06760 | late embryogenesis abundant protein LEA like | 11.3 | 1.9 |
| 257876_at | At3g17130 | hypothetical protein | 11.3 | 2.3 |
| 260727_at | At1g48100 | polygalacturonase, putative | 11.3 | 2.3 |
| 246125_at | At5g19875 | Expressed protein | 10.6 | 2.0 |
| 251603 at | At3g57760 | putative protein | 10.6 | 1.1 |
| 256017_at | Atlg19180 | expressed protein | 10.6 | 1.5 |
| 264617_at | At2g17660 | unknown protein | 10.6 | ND |
| 264787_at | At2g17840 | putative senescence-associated protein 12 | 10.6 | 3.5 |
| 245757_at | At1g35140 | phosphate-induced (phi-1) protein, putative | 9.8 | 1.6 |
| 252346_at | At3g48650 | hypothetical protein | 9.8 | 2.0 |
| 253643_at | At4g29780 | expressed protein | 9.8 | 3.2 |
| 254667_at | At4g18280 | glycine-rich cell wall protcin-like | 9.8 | 1.2 |
| 264389_at | At1g11960 | unknown protein | 9.8 | 1.7 |
| 266545_at | At2g35290 | hypothetical protein | 9.8 | 1.4 |
| 266720_s_at | At2g46790 | expressed protein | 9.8 | 4.9 |
| 245251_at | At4g17615 | calcineurin B-like protein 1 | 9.2 | 3.0 |
| 247431_at | At5g62520 | putative protein | 9.2 | 1.5 |
| 248964_at | At5g45340 | cytochrome p450 family | 9.2 | 3.0 |
| 252368_at | At3g48520 | cytochrome p450, putative | 9.2 | 1.3 |
| 262164_at | At1g78070 | expressed protein | 9.2 | 4.3 |
| 252102_at | At3g50970 | dehydrin Xcro2 | 8.6 | 4.0 |
| 262359_at | At1g73070 | leucine rich repeat protein family | 8.6 | ND |
| 262731_at | At1g16420 | hypothetical protein common family | 8.6 | 2.8 |
| 245677_at | At1g56660 | hypothetical protein | 8.0 | 2.8 |
| 245734_at | At1g73480 | lysophospholipase homolog, putative | 8.0 | 2.6 |
| 247177_at | At5g65300 | expressed protein | 8.0 | 3.7 |
| 250053_at | At5g17850 | potassium-dependent sodium-calcium exchanger - like protein | 8.0 | 2.0 |
| 254120_at | At4g24570 | mitochondrial carrier protein family | 8.0 | 3.0 |
| 254926_at | At4g11280 | ACC synthase (AtACS-6) | 8.0 | 2.3 |
| 263789_at | At2g24560 | putative GDSL-motif lipase/hydrolase | 8.0 | ND |
| 245346_at | At4g17090 | glycosyl hydrolase family 14 (beta-amylase) | 7.5 | 2.6 |
| 253425_at | At4g32190 | putative protein | 7.5 | 3.2 |
| 254085_at | At4g24960 | abscisic acid-induced - like protein | 7.5 | 2.3 |
| 259076_at | At3g02140 | expressed protein | 7.5 | 1.1 |
| 260227_at | At1g74450 | expressed protein | 7.5 | 2.0 |
| 260915_at | At1g02660 | expressed protein | 7.5 | 1.7 |
| 262677_at | At1g75860 | unknown protein | 7.5 | 2.6 |
| 266532_at | At2g16890 | putative glucosyltransferase | 7.5 | 3.0 |
| 247925_at | At5g57560 | xyloglucan endotransglycosylase (TCH4) | 7.0 | 2.1 |
| 252563_at | At3g45970 | putative protein | 7.0 | 1.2 |
| 254850_at | At4g12000 | putative protein | 7.0 | 2.3 |
| 260744_at | At1g15010 | expressed protein | 7.0 | 1.7 |
| 263931_at | At2g36220 | expressed protein | 7.0 | 3.0 |
| 245306_at | At4g14690 | Expressed protein | 6.5 | 2.6 |
| 246495_at | At5g16200 | putative protein | 6.5 | 1.7 |
| 248870_at | At5g46710 | putative protein | 6.5 | 2.6 |
| 253292_at | At4g33985 | Expressed protein | 6.5 | 2.0 |
| 253872_at | At4g27410 | putative protein | 6.5 | 1.5 |
| 258792_at | At3g04640 | expressed protein | 6.5 | 3.0 |
| 259516_at | At1g20450 | expressed protein | 6.5 | 3.2 |
| 262050_at | At1g80130 | expressed protein | 6.5 | 1.2 |
| 245427_at | At4g17550 | putative protein | 6.1 | 1.2 |
| 253859_at | At4g27657 | Expressed protein | 6.1 | ND |
| 261187_at | At1g32860 | glycosyl hydrolase family 17 | 6.1 | 1.4 |
| 262448_at | At1g49450 | En/Spm-like transposon protein, putative | 6.1 | ND |
| 266757_at | At2g46940 | unknown protein | 6.1 | 1.3 |
| 252131_at | At3g50930 | BCS1 protein-like protein | 5.7 | 1.6 |
| 255795_at | At2g33380 | RD20 protein | 5.7 | -1.3 |
| 258321_at | At3g22840 | early light-induced protein | 5.7 | 2.3 |
| 262496_at | At1g21790 | expressed protein | 5.7 | 2.0 |
| 265119_at | At1g62570 | flavin-containing monooxygenase, putative | 5.7 | 2.0 |
| 246018_at | At5g10695 | Expressed protein | 5.3 | 1.7 |
| 248820_at | At5g47060 | putative protein | 5.3 | 1.7 |
| 249918_at | At5g19240 | putative protein | 5.3 | 1.9 |
| 253830_at | At4g27652 | Expressed protein | 5.3 | 1.7 |
| 246490_at | At5g15950 | S-adenosylmethionine decarboxylase (adoMetDC2) | 4.9 | 2.1 |
| 253284_at | At4g34150 | putative protein | 4.9 | 1.9 |
| 253323_at | At4g33920 | putative protein | 4.9 | 2.5 |
| 253614_at | At4g30350 | putative protein | 4.9 | 1.5 |
| 264655_at | At1g09070 | expressed protein | 4.9 | 1.9 |
| 245533_at | At4g15130 | putative phosphocholine cytidylyltransferase | 4.6 | 2.1 |
| 246831_at | At5g26340 | hexose transporter - like protein | 4.6 | 2.0 |
| 247137_at | At5g66210 | calcium-dependent protein kinase | 4.6 | 1.4 |
| 247226_at | At5g65100 | putative protein | 4.6 | ND |
| 250467_at | At5g10100 | trehalose-6-phosphate phosphatase -like protein | 4.6 | ND |
| 252414_at | At3g47420 | putative protein | 4.6 | 1.9 |
| 252997_at | At4g38400 | putative pollen allergen | 4.6 | 1.1 |
| 253595_at | At4g30830 | putative protein | 4.6 | ND |
| 253832_at | At4g27654 | Expressed protein | 4.6 | 1.3 |
| 258188_at | At3g17800 | expressed protein | 4.6 | 1.4 |
| 259479_at | At1g19020 | Expressed protein | 4.6 | 1.7 |
| 261405_at | At1g18740 | expressed protein | 4.6 | 2.0 |
| 262881_at | At1g64890 | expressed protein | 4.6 | 2.1 |
| 264000_at | At2g22500 | mitochondrial carrier protein family | 4.6 | 2.0 |
| 265668_at | At2g32020 | putative alanine acetyl transferase | 4.6 | 2.3 |
| 265797_at | At2g35715 | Expressed protein | 4.6 | ND |
| 248686_at | At5g48540 | 33 kDa secretory protein-like | 4.3 | 1.6 |
| 250676_at | At5g06320 | harpin-induced protein-like | 4.3 | 1.6 |
| 251259_at | At3g62260 | protein phosphatase 2C (PP2C) | 4.3 | 2.1 |
| 254300_at | At4g22780 | Translation factor EF-1 alpha - like protein | 4.3 | -1.1 |
| 261356_at | At1g79660 | unknown protein | 4.3 | 1.6 |
| 264636_at | At1g65490 | expressed protein | 4.3 | 1.4 |
| 246468_at | At5g17050 | UDP glucosc:flavonoid 3-o-glucosyltransferase -like protein | 4.0 | 2.0 |
| 248607 _at | At5g49480 | NaCl-inducible Ca2+-binding protein-like; calmodulin-like | 4.0 | 1.5 |
| 250279_at | At5g13200 | ABA-responsive protein - like | 4.0 | 1.2 |
| 252053_at | At3g52400 | syntaxin SYP122 | 4.0 | 1.9 |
| 256633_at | At3g28340 | unknown protein | 4.0 | 2.0 |
| 258207_at | At3g14050 | putative GTP pyrophosphokinase | 4.0 | 1.7 |
| 258805_at | A13g04010 | glycosyl hydrolase family 17 | 4.0 | 1.3 |
| 261912_s_at | At1g66000 | hypothetical protein | 4.0 | ND |
| 264989_at | At1g27200 | expressed protein | 4.0 | 1.6 |
| 265276_at | At2g28400 | hypothetical protein | 4.0 | -1.1 |
| 267261_at | At2g23120 | expressed protein | 4.0 | 1.7 |
| 247693_at | At5g59730 | putative protein | 3.7 | 1.9 |
| 253113_at | At4g35985 | putative protein | 3.7 | 1.4 |
| 253165_at | At4g35320 | putative protein | 3.7 | 1.9 |
| 253879 _s_at | At4g27570 | UDP rhamnose--anthocyanidin-3-glucoside rhamnosyltransferase - like protein | 3.7 | 1.2 |
| 253915 at | At4g27280 | putative protein | 3.7 | 1.9 |
| 259426_at | At1g01470 | hypothetical protein | 3.7 | 1.6 |
| 259445_at | At1g02400 | dioxygenase, putative | 3.7 | 1.9 |
| 260410_at | At1g69870 | putative peptide transporter | 3.7 | 1.1 |
| 261581 at | At1g01140 | serine threonine kinase, putative | 3.7 | 1.5 |
| 262113_at | At1g02820 | late embryogenis abundant protein, putative | 3.7 | 1.1 |
| 262382_at | At1g72920 | disease resistance protein (TIR-NBS class), putative | 3.7 | 1.9 |
| 265665_at | At2g27420 | cysteine proteinase | 3.7 | 1.0 |
| 267069_at | At2g41010 | unknown protein | 3.7 | 1.2 |
| 245450_at | At4g16880 | disease resistance RPP5 like protein (fragment) | 3.5 | ND |
| 246289_at | At3g56880 | putative protein | 3.5 | 1.3 |
| 249204_at | At5g42570 | expressed protein | 3.5 | 1.5 |
| 249622_at | At5g37550 | putative protein | 3.5 | 1.4 |
| 250335_at | At5g11650 | lysophospholipase - like protein | 3.5 | 1.7 |
| 251372_at | At3g60520 | putative protein | 3.5 | 1.5 |
| 254707_at | At4g18010 | putative protein | 3.5 | 1.1 |
| 257154_at | At3g27210 | expressed protein | 3.5 | 1.5 |
| 259705_at | At1g77450 | GRABI-like protein | 3.5 | 1.3 |
| 261037_at | At1g17420 | lipoxygenase | 3.5 | 1.2 |
| 261937_at | At1g22570 | peptide transporter, putative | 3.5 | 1.6 |
| 264024_at | At2g21180 | expressed protein | 3.5 | 1.1 |
| 264458_at | At1g10410 | unknown protein | 3.5 | 1.2 |
| 266799_at | At2g22860 | unknown protein | 3.5 | 1.4 |
| 247280_at | At5g64260 | phi-1-like protein | 3.2 | 1.6 |
| 251356_at | At3g61060 | putative protein | 3.2 | 1.5 |
| 252316_at | At3g48700 | putative protein | 3.2 | 1.3 |
| 253824_at | At4g27940 | putative protein | 3.2 | 1.1 |
| 256526 at | At1g66090 | disease resistance protein (TIR-NBS class), putative | 3.2 | 1.4 |
| 256595_x _at | At3g28530 | hypothetical protein | 3.2 | 1.1 |
| 265648_at | At2g27500 | glycosyl hydrolase family 17 | 3.2 | 1.1 |
| 266097 at | At2g37970 | expressed protein | 3.2 | 1.6 |
| 267335 s_at | At2g19440 | glycosyl hydrolase family 17 | 3.2 | 1.6 |
| 245699_at | At5g04250 | putative protein | 3.0 | 1.2 |
| 247467_at | At5g62130 | putative protein | 3.0 | 1.5 |
| 249583_at | At5g37770 | CALMODULIN-RELATED PROTEIN 2, TOUCH-INDUCED (TCH2) | 3.0 | 1.1 |
| 249626_at | At5g37540 | putative protein | 3.0 | 1.2 |
| 252474_at | At3g46620 | putative protein | 3.0 | 1.3 |
| 253628_at | At4g30280 | xyloglucan endotransglycosylase, putative | 3.0 | 1.4 |
| 253835_at | At4g27820 | glycosyl hydrolase family 1 | 3.0 | 1.2 |
| 254158_at | At4g24380 | putative protein | 3.0 | 1.4 |
| 254188_at | At4g23920 | UDPglucose 4-epimerase - like protein | 3.0 | 1.2 |
| 254634_at | At4g18650 | putative protein | 3.0 | ND |
| 254973_at | At4g10460 | putative retrotransposon | 3.0 | ND |
| 256763_at | At3g16860 | unknown protein | 3.0 | 1.0 |
| 257519_at | At3g01210 | RRM-containing protein | 3.0 | -1.1 |
| 258894_at | At3g05650 | disease resistance protein family | 3.0 | 1.4 |
| 265841_at | At2g35710 | putative glycogenin | 3.0 | 1.5 |
| 266271_at | At2g29440 | glutathione transferase, putative | 3.0 | 1.2 |
| 266316_at | At2g27080 | expressed protein | 3.0 | 1.1 |
| 267631_at | At2g42150 | hypothetical protein | 3.0 | 1.1 |

**Table 1B: Cold-responsive genes with similar induction in the wild type and ice1**

| Probe Set | AGI ID | Gene Title | Fold Change | |
|---|---|---|---|---|
| | | | WT | *ice1* |
| 254075_at | At4g25470 | **CBF2/DREB1C** | 104.0 | 137.2 |
| 261263_at | At1g26790 | Dof zinc finger protein | 68.6 | 55.7 |
| 257262_at | At3g21890 | CONSTANS B-box zinc finger family protein | 7.5 | 5.3 |
| 259834_at | At1g69570 | Dof zinc finger protein | 7.0 | 5.7 |
| 256430_at | At3g11020 | DREB2B | 6.1 | 4.9 |
| 248389_at | At5g51990 | DRE binding protein | 5.3 | 4.0 |
| 257053_at | At3g15210 | AtERF4 | 5.3 | 2.8 |
| 248744_at | At5g48250 | CONSTANS B-box zinc finger family protein | 4.9 | 3.0 |
| 249606_at | At5g37260 | CCA1, putative | 4.9 | 9.2 |
| 267028 _at | At2g38470 | WRKY family transcription factor | 4.9 | 3.0 |
| 246523_at | At5g15850 | CONSTANS-LIKE 1 | 4.0 | 3.2 |
| 248799_at | At5g47230 | AtERF5 | 4.0 | 3.5 |
| 247452_at | At5g62430 | Dof zinc finger protein | 3.7 | 3.7 |
| 251190_at | At3g62690 | RING-H2 zinc finger protein ATL5 | 3.7 | 2.1 |
| 253722_at | At4g29190 | Zn finger protein, putative | 3.7 | 4.6 |
| 259992_at | At1g67970 | putative heat shock transcription factor | 3.7 | 2.3 |
| 263252_at | At2g31380 | CONSTANS-like B-box zinc finger protein | 3.7 | 3.7 |
| 263739_at | At2g21320 | CONSTANS B-box zinc finger family protein | 3.7 | 2.3 |
| 252429 _at | At3g47500 | Dof zinc finger protein | 3.5 | 4.3 |
| 253140_at | At4g35480 | RING-H2 finger protein RHA3b | 3.5 | 2.0 |
| 258742_at | At3g05800 | bHLH protein | 3.5 | 6.5 |
| 265939 _at | At2g19650 | CHP-rich zinc finger protein, putative | 3.5 | 2.8 |
| 249415_at | At5g39660 | Dof zinc finger protein | 3.2 | 3.7 |
| 259364_at | At1g13260 | DNA-binding protein (RAV1) | 3.2 | 2.1 |
| 262590_at | At1g15100 | putative RING-H2 zinc finger protein | 3.2 | 2.0 |
| 263823_s_at | At2g40350 | AP2 domain transcription factor | 3.0 | 5.7 |
| 264511_at | At1g09350 | putative galactinol synthase | 17.1 | 12.1 |
| 264314_at | At1g70420 | expressed protein | 13.0 | 9.8 |
| 247478_at | At5g62360 | DC1.2 homologue - like protein | 11.3 | 11.3 |
| 253322_at | At4g33980 | putative protein | 11.3 | 8.0 |
| 249741_at | At5g24470 | putative protein | 8.0 | 6.5 |
| 247047_at | At5g66650 | putative protein | 7.0 | 4.3 |
| 263495_at | At2g42530 | COR15b | 6.5 | 9.8 |
| 265536_at | At2g15880 | unknown protein | 6.5 | 5.3 |
| 249174_at | At5g42900 | putative protein | 6.1 | 3.5 |
| 249191_at | At5g42760 | putative protein | 6.1 | 4.3 |
| 264153_at | At1g65390 | disease resistance protein (TIR class), putative | 6.1 | 4.9 |
| 250099_at | At5g17300 | expressed protein | 5.7 | 7.5 |
| 265725_at | At2g32030 | putative alanine acetyl transferase | 5.7 | 3.0 |
| 246922_at | At5g25110 | serine/threonine protein kinase-like protein | 4.9 | 4.9 |
| 251494_at | At3g59350 | protein kinase-like protein | 4.9 | 2.8 |
| 246821_at | At5g26920 | calmodulin-binding protein | 4.6 | 2.6 |
| 255733_at | At1g25400 | expressed protein | 4.6 | 3.0 |
| 257650_at | At3g16800 | protein phosphatase 2C (PP2C) | 4.6 | 2.5 |
| 266832_at | At2g30040 | putative protein kinase | 4.6 | 2.8 |
| 267357_at | At2g40000 | putative nematode-resistance protein | 4.6 | 3.7 |
| 249411_at | At5g40390 | glycosyl hydrolase family 36 | 4.3 | 3.5 |
| 256266_at | At3g12320 | expressed protein | 4.3 | 4.0 |
| 252956_at | At4g38580 | copper chaperone (CCH)-related | 4.0 | 2.3 |
| 253455_at | At4g32020 | putative protein | 4.0 | 2.6 |
| 259570_at | At1g20440 | hypothetical protein | 4.0 | 2.3 |
| 262383_at | At1g72940 | disease resistance protein (TIR-NBS class), putative | 4.0 | 2.1 |
| 247393_at | At5g63130 | unknown protein | 3.7 | 3.2 |
| 252661_at | At3g44450 | putative protein | 3.7 | 2.5 |
| 259990_s_at | At1g68050 | F-box protein FKF1/ADO3. AtFBX2a | 3.7 | 2.3 |
| 264213_at | At1g65400 | hypothetical protein | 3.7 | 2.0 |
| 245777_at | At1g73540 | unknown protein | 3.5 | 2.5 |
| 248745_at | At5g48260 | unknown protein | 3.5 | 2.5 |
| 248846_at | At5g46500 | putative protein | 3.5 | 2.6 |
| 249063_at | At5g44110 | ABC transporter family protein | 3.5 | 2.1 |
| 257654_at | At3g13310 | DnaJ protein, putative | 3.5 | 2.1 |
| 257925_at | At3g23170 | expressed protein | 3.5 | 1.9 |
| 261048_at | At1g01420 | flavonol 3-o-glucosyltransferase, putative | 3.5 | 2.0 |
| 263216_s_at | At1g30720 | FAD-linked oxidoreductase family | 3.5 | 2.3 |
| 265184_at | At1g23710 | expressed protein | 3.5 | 2.3 |
| 245558_at | At4g15430 | hypothetical protein | 3.2 | 3.5 |
| 248164_at | At5g54490 | putative protein | 3.2 | 2.5 |
| 248502_at | At5g50450 | putative protein | 3.2 | 4.3 |
| 252010_at | At3g52740 | expressed protein | 3.2 | 2.5 |
| 253679_at | At4g29610 | cytidine deaminase 6 (CDA6) | 3.2 | 2.0 |
| 256548_at | At3g14770 | expressed protein | 3.2 | 1.9 |
| 256577_at | At3g28220 | unknown protein | 3.2 | 2.3 |
| 257083_s_at | At3g20590 | non-race specific disease resistance protein, putative | 3.2 | 2.3 |
| 260046_at | At1g73800 | Expressed protein | 3.2 | 2.0 |
| 261958_at | At1g64500 | peptide transporter, putative | 3.2 | 2.6 |
| 263352_at | At2g22080 | En/Spm-like transposon protein | 3.2 | 1.9 |
| 263452_at | At2g22190 | putative trehalose-6-phosphate phosphatase | 3.2 | 2.3 |
| 265093_at | At1g03905 | ABC transporter family protein | 3.2 | 1.7 |
| 267293_at | At2g23810 | hypothetical protein | 3.2 | 1.7 |
| 245119_at | At2g41640 | expressed protein | 3.0 | 2.6 |
| 246270_at | At4g36500 | putative protein | 3.0 | 3.0 |
| 247793_at | At5g58650 | putative protein | 3.0 | 1.7 |
| 256442_at | At3g10930 | expressed protein | 3.0 | 1.9 |
| 256487_at | At1g31540 | disease resistance protein (TIR-NBS-LRR class), putative | 3.0 | 2.1 |
| 259428_at | At1g01560 | MAP kinase, putative | 3.0 | 1.7 |
| 266834_s_at | At2g30020 | protein phosphatase 2C (PP2C) | 3.0 | 2.6 |
| 267364_at | At2g40080 | expressed protein | 3.0 | 2.3 |

**Table 1C: Cold responsive genes with higher induction in ice1**

| Probe Set | AGI ID | Gene Title | Fold Change | |
|---|---|---|---|---|
| | | | WT | *ice1* |
| 261248_at | At1g20030 | calreticulin, putative | 4.6 | 13.9 |
| 258383_at | At3g15440 | hypothetical protein | 4.3 | 9.2 |

### The ice1 mutation impairs chilling and freezing tolerance

At normal growth temperatures, *ice1* and wild type seedlings were similar in size (Fig. 2A). Although adult *ice1* plants were smaller, they were not very different from the wild type in flowering time and fertility (Fig. 2B). Ten-day-old seedlings of *ice1* and wild type grown on separate halves of the same agar plates were cold acclimated at 4°C for four days and then subjected to a freezing tolerance assay. The *ice1* mutant was less freezing-tolerant than the wild type at all freezing temperatures (Figs. 2C and 2D). Freezing at -10°C for 2 hours killed about 50% of *ice1* mutant plants whereas less than 20% of wild type plants were killed at this temperature (Fig. 2D). When newly germinated (at 22° C) *ice1* and wild type seedlings were transferred to 4°C (with 30 ± 2 µmol quanta. m⁻².s⁻¹ light), chilling injury became apparent in the mutant after 4 weeks of cold treatment (Fig. 2E). After 6 weeks of chilling stress, 100% of wild type but only 20% of *ice1* mutant plants survived (Fig. 2F).

### Positional cloning of ICE1

To map the *ice1* mutation, a homozygous *ice1* mutant in the *CBF3-LUC* Columbia background was crossed to wild type plants of the Ler ecotype. F1 plants from the cross were selfed to produce F2 seeds. Since the *ice1* mutation is dominant, we selected from the segregating F2 population seedlings with the wild type phenotype (based on plant size and morphology) for mapping. A total of 662 wild type plants were selected and used for mapping with simple sequence length polymorphism and cleaved amplified polymorphic sequence markers (see Materials and Methods section for details), which initially placed *ice1* on the middle of chromosome 3, then narrowed its location to a 58 kb region on the MLJ 15 and MDJ14 BAC clones. Candidate genes in this region were amplified from homozygous *ice1* mutant plants and sequenced. The sequences were compared with the published sequence of *Arabidopsis* ecotype Columbia and a single G to A mutation in the hypothetical MLJ15.14 gene was found.

To confirm that MLJ15.14. is the *ICE1* gene, the MLJ15.14 gene including 2,583 bp upstream of the initiation codon and 615 bp downstream of the stop codon was cloned from *ice1* mutant plants. This fragment was inserted into a binary vector and introduced into *CBF3-LUC* Columbia wild type plants by *Agrobacterium*-mediated transformation. Transgenic plants were selected based on their hygromycin resistance, and cold-induced bioluminescence in the T2 lines was compared with that of the wild type. The MLJ15.14 gene from *ice1* suppressed cold-induced luminescence from the wild type plants (Figs. 3A and 3B) and reduced the plant height to that of *ice1* mutant, thus confirming that MIJ15.14 is *ICE1.*

### ICE1 encodes a constitutively expressed and nuclear localized MYC-like basic helix-loop-helix transcription factor

The open reading frame of *ICE1* (SEQ ID NO: 1)was determined by sequencing cDNAs obtained by RT-PCR. The open reading frame was determined to be:

*ICE1* is predicted to encode a protein of 494 amino acids, with an estimated molecular mass of 53.5 kDa as follows (SEQ ID NO: 2):

Database searches revealed that ICE1 contains a MYC-like basic helix-loop-helix (bHLH) domain at its C-terminal half (Figs. 4A and 4B). Over the entire length of the protein, ICE1 shows amino acid sequence similarity to an unknown protein of *Arabidopsis* (At1g12860). The *icel* mutation changes Arg236, conserved in these two *Arabidopsis* proteins, to His. The bHLH domain of *ICE1* shows high amino acid similarity to that of known MYC-related bHLH transcription factors (Fig. 4B). All MYC binding promoter elements contain the CA nucleotides that are contacted by a conserved glutamic acid in the bHLH zipper domain (Grandori et al., 2000). This glutamic acid residue (Glu312) is also conserved in the basic DNA binding domain of *ICE1* (Fig. 4B). An acidic domain near the amino terminus characterizes the bHLH family of transcription factors and a conserved bHLH DNA binding and dimerization domain near the carboxyl terminus (Purugganan and Wessler 1994). All these features are present in *ICE1* protein (Fig. 4A).

To analyze the expression pattern of *ICE1* in different tissues, T2 lines of transgenic *Arabidopsis* plants expressing an *ICE1* promoter-GUS transgene were analyzed. GUS expression was detected in roots, leaves, stem and floral parts. Semi-quantitative RT-PCR analysis also showed that *ICE1* was expressed constitutively and the expression was stronger in leaves and stems than in other tissues (Figs. 5A and 5B). RNA blot analysis showed that the *ICE1* transcript was slightly up-regulated by cold, NaCl and ABA but not by dehydration (Fig. 5C).

To examine the subcellular localization of the ICE1 protein, ICE1 was fused in-frame to the C-terminal side of the green fluorescent protein (GFP) and expressed under control of the CaMV 35S promoter. Confocal imaging of GFP fluorescence in T2 transgenic plants showed that the GFP-ICE1 fusion protein is present in the nucleus under either warm (Fig. 5D) or cold temperatures.

### ICE1 binds to MYC recognition sites in the CBF3 promoter

ICE1 has a basic helix-loop-helix (bHLH) domain and its amino acid sequence in the basic region is highly conserved with other bHLH proteins (Fig. 4B), and therefore may recognize promoter elements similar to the DNA-binding sites for known bHLH proteins. These proteins recognize DNA with the consensus sequence CANNTG (Meshi and Iwabuchi 1995). In the promoter region of *CBF3,* there are five potential MYC-recognition elements within a 1 kb region upstream of the transcription initiation site (Shinwari et al. 1998). These possible MYC-recognition sites, designated MYC-1 through MYC-5, fall into four groups because MYC-3 and MYC-5 share the same consensus sequence, CATTTG (Fig. 6A). Thus, MYC-3 was used to represent both MYC-3 and MYC-5. To determine whether ICE1 binds to these MYC-recognition sites in the *CBF3* promoter, we expressed and purified His-ICE 1 fusion protein from *E. coli.* Four DNA fragments encompassing each possible MYC-recognition site were used for interaction with His-ICE1 in an electrophoresis mobility shift assay (EMSA).

Several complexes were observed when ICE1 was incubated with any of the four DNA fragments (MYC-1 through MYC-4), indicating that ICE1 is able to bind to these sequences (Fig. 6B). The MYC-2 fragment formed one major complex with ICE1, while the other DNA fragments formed several complexes with ICE1. These complexes were abolished by the addition of increasing amounts of cold competitors with the same sequences, but not by P1 or P2, which contains a putative MYB- recognition site and a non-related sequence, respectively (Fig. 6B). This specificity of competition strengthens the hypothesis that the interaction between DNA and ICE1 requires the MYC-recognition sequences. When the MYC-2 fragment was used as a probe, the complex was most efficiently competed off by the cold MYC-2 competitor, suggesting that ICE1 has a higher affinity for the MYC-2 site than for the other sites (Fig. 6C). The complex formed by ICE1 and the MYC-2 fragment was less affected by a mutated competitor than by the wild type competitor (Fig. 6D). Together, these results show that ICE1 interacts specifically with the MYC-recognition sites in the *CBF3* promoter. The *ice1* mutation does not appear to affect ICE1 interaction with the *CBF3* promoter, because the Arg236 to His mutant form of ICE 1 was also able to bind to the MYC-2 probe (Fig. 6E).

### ICEI is a transcriptional activator that positively regulates CBF expression

Transient expression assays were carried out to determine whether *ICE1* acts as a transcriptional activator or repressor. An effector plasmid was constructed by fusing *ICE1* with the DNA binding domain of the yeast GAL4 transcriptional activator (*GAL4-ICE1,* Fig. 7A). When the wild type *GAL4-ICE1* and a *GAL4*-responsive reporter gene, *GAL4-LUC,* were delivered into *Arabidopsis* leaves by particle bombardment, the luciferase activity increased 20 fold relative to the control with or without an effector plasmid containing only the GAL4 DNA binding domain (Fig. 7B). The Arg236 to His mutant form of *GAL4-ICE1* also activated the *GAL4*-responsive transcription (Fig. 7B). These results suggest that ICE 1 is a transcriptional activator, and that the *ice1* mutation does not affect the function of the transcriptional activation domain.

A null allele of *ice1* created by T-DNA insertion does not show any phenotypes of the dominant *ice1* mutant, suggesting that there is functional redundancy in the *ICE1* gene family. We overexpressed *ICE1* in wild type *Arabidopsis* plants by using the strong constitutive super promoter. None of the overexpression lines showed any *ice1* mutant phenotypes. RNA blot analysis showed that *ICE1*-overexpression did not activate *CBF3* expression at warm temperatures. However, *ICE1*-overexpression enhanced the expression of the endogenous *CBF3* gene as well as the *CBF3-LUC* reporter gene in the cold (Figs. 7C and 7D). Cold-induction of *CBF2, RD29A* and *COR15A* was also enhanced in the *Super-ICE1* transgenic plants (Fig. 7C). When the *Super-ICE1* transgenic plants and wild type control plants in the same agar plates were cold acclimated at 4°C for 5 days and then subjected to freezing treatment at -8°C for 4 hours, the *ICE1* overexpression transgenic seedlings showed a higher survival rate (75.9±6.5%) than that of control plants (37.2±12.6%) (Fig. 7E). The *ICE1* overexpression transgenic plants did not exhibit obvious growth or developmental abnormalities. These results suggest that ICE1 is a positive regulator of *CBF3,* and that the dominant nature of *ice1* is likely caused by a dominant negative effect of the mutation.

### REFERENCES

Browse, J. and Xin, Z. 2001. Temperature sensing and cold acclimation. Curr. Opin. Plant Biol. 4: 241-246.
Chinnusamy, V., Stevenson, B., Lee, B.-h., and Zhu, J.-K. 2002. Screening for gene regulation mutants by bioluminescence imaging. Science's STKE http://stke.sciencemag.org/cgi/content/full/sigtrans;2002/140/p110 (9 Jul 2002).
Fowler, S., and Thomashow, M. F. 2002. Arabidopsis transcriptome profiling indicates that multiple regulatory pathways are activated during cold acclimation in addition to the CBF cold response pathway. Plant Cell 14: 1675-1690.
Gilmour, S. J., Artus, N. N., and Thomashow, M. F. 1992. cDNA sequence analysis and expression of two cold regulated genes of Arabidopsis thaliana. Plant Mol. Biol. 18: 13-32.
Gilmour, S.J., Zarka, D.G., Stockinger, E.J., Salazar, M.P., Houghton, J.M., and Thomashow, M.F. 1998. Low temperature regulation of the Arabidopsis CBF family of AP2 transcriptional activators as an early step in cold-induced COR gene expression. Plant J. 16: 433-442.
Gong, Z., Lee, H., Xiong, L., Jagendorf, A., Stevenson, B., and Zhu, J.-K. 2002. RNA helicase-like protein as an early regulator of transcription factors for plant chilling and freezing tolerance. Proc. Natl. Acad. Sci. USA 99: 11507-11512.
Grandori, C., Cowley, S.M., James, L.P. and Eisenman, R.N. 2000. The MYC/MAX/MAD network and the transcriptional control of cell behavior. Annu. Rev. Cell. Dev. Biol. 16: 653-699.
Guo, Y., Xiong, L., Ishitani, M. and Zhu, J.-K. 2002. An Arabidopsis mutation in translation elongation factor 2 causes superinduction of CBF/DREB1 transcription factor genes but blocks the induction of their downstream targets under low temperature. Proc. Natl. Acad. Sci. USA. 99: 7786-7791.
Guy, C.L. 1990. Cold acclimation and freezing stress tolerance: role of protein metabolism. Annu. Rev. Plant Physiol. Plant Mol. Biol. 41: 187-223.
Hao, D., Ohme-Takagi, M., and Sarai, A. 1998. Unique mode of GCC box recognition by the DNA-binding domain of ethylene-responsive element-binding factor (ERF domain) in plant. J. Biol. Chem. 273: 26857-26861.
Hsieh, T.H., Lee, J.T., Yang, P.T., Chiu, L.H., Charng, Y.Y., Wang, Y.C., and Chan, M.T. 2002. Heterology expression of the Arabidopsis C-repeat/dehydration response element binding factor 1 gene confers elevated tolerance to chilling and oxidative stresses in transgenic tomato. Plant Physiol. 129: 1086-1094.
Hughes, M. and Dunn, M. 1996. The molecular biology of plant acclimation to low temperature. J. Exp. Bot. 47: 291-305.
Ishitani, M., Xiong, L., Lee, H., Stevenson, B., and Zhu, J.K. 1998. HOS1, a genetic locus involved in cold-responsive gene expression in Arabidopsis. Plant Cell 10: 1151-1161.
Ishitani, M., Xiong, L., Stevenson, B., and Zhu, J.K. 1997. Genetic analysis of osmotic and cold stress signal transduction in Arabidopsis: interactions and convergence of abscisic acid-dependent and abscisic acid-independent pathways. Plant Cell 9: 1935-1949.
Jaglo-Ottosen, K.R., Gilmour, S.J., Zarka, D.G., Schabenberger, O., and Thomashow, M.F. 1998. Arabidopsis CBF1 overexpression induces cor genes and enhances freezing tolerance. Science 280: 104-106.
Knight, H., Veale, E.L., Warren, G.J., and Knight, M.R. 1999. The sfr6 mutation in Arabidopsis suppresses low-temperature induction of genes dependent on the CRT/DRE sequence motif. Plant Cell 11: 875-886.
Kurkela, S., and Franck, M. 1990. Cloning and characterization of a cold- and ABA-inducible Arabidopsis gene. Plant Mol. Biol. 15: 137-144.
Lee, H., Guo, Y., Ohta, M., Xiong, L., Stevenson, B., and Zhu, J.-K. 2002. LOS2, a genetic locus required for cold-responsive gene transcription encodes a bifunctional enolase. EMBO. J. 21: 2692-2702.
Lee, H., Xiong, L., Gong, Z., Ishitani, M., Stevenson, B., and Zhu, J.K. 2001. The Arabidopsis HOS1 gene negatively regulates cold signal transduction and encodes a RING finger protein that displays cold-regulated nucleo-cytoplasmic partitioning. Genes Dev. 15: 912-924.
Li, X. , Gong, Z. , Koiwa, H. , Niu, X. , Espartero, J. , Zhu, X. , Veronese, P. , Ruggiero, B. , Bressan, R. , Weller, S. C., and Hasegawa, P. M. 2001. Bar-expressing peppermint (Mentha × Piperita L. var. Black Mitcham) plants are highly resistant to the glufosinate herbicide Liberty. Mol. Breed. 8: 109-118.
Lin, C., and Thomashow, M. F. 1992. DNA sequence analysis of a complementary DNA for cold-regulated Arabidopsis gene cor15 and characterization of the COR15 polypeptide. Plant Physiol. 99: 519-525.
Liu, J., and Zhu, J.-K. 1997. Proline accumulation and salt-stress-induced gene expression in a salt-hypersensitive mutant of Arabidopsis. Plant Physiol. 114: 591-596.
Liu, Q., Sakuma, Y., Abe, H., Kasuga, M., Miura, S., Yamaguchi-Shinozaki, K., and Shinozaki, K. 1998. Two transcription factors, DREB 1 and DREB2, with an EREBP/AP2 DNA binding domain, separate two cellular signal transduction pathways in drought- and low temperature-responsive gene expression, respectively, in Arabidopsis. Plant Cell 10: 1391-1406.
Liu, L., White, M.J., and MacRae, T.H. 1999. Transcription factors and their genes in their genes in higher plants: Functional domains, evolution and regulation. Eur. J. Biochem. 262: 247-257.
Meshi, T., and Iwabuchi, M. 1995. Plant transcription factors. Plant Cell. Physiol. 36: 1405-1420.
Mohapatra, S.S., Wolfraim, L., Poole, R.J., and Dhindsa, R.S. 1989. Molecular cloning and relationship to freezing tolerance of cold-acclimation-specific genes of alfalfa. Plant Physiol. 89: 375-380.
Ohta, M., Ohme-Takagi, M., and Shinshi, H. 2000. Three ethylene-responsive transcription factors in tobacco with distinct transactivation functions. Plant J. 22: 29-38.
Ohta, M., Matsui, K., Hiratsu, K., Shinshi, H., and Ohme-Takagi, M. 2001. Repression Domains of Class II ERF transcriptional repressors share an essential motif for active repression. Plant Cell 13: 1959-1968.
Purugganan, M.D., and Wessler, S.R. 1994. Molecular evolution of the plant R regulatory gene family. Genetics 138: 849-854.
Shinwari, Z.K., Nakashima, K., Miura, S., Kasuga, M., Seki, M., Yamaguchi-Shinozaki, K., and Shinozaki, K. 1998. An Arabidopsis gene family encoding DRE/CRT binding proteins involved in low-temperature-responsive gene expression. Biochem. Biophys. Res. Commun. 250: 161-170.
Spelt, C., Quattrocchio, F., Mol, J.N.M., and Koes, R. 2000. Anthocyanin1 of petunia encodes a basic-helix-loop-helix protein that directly activates transcription of structural anthocyanin genes. Plant Cell 12: 1619-1631.
Stockinger, E.J. Gilmour, S.J., and Thomashow, M.F. 1997. Arabidopsis thaliana CBF1 encodes an AP2 domain-containing transcription activator that binds to the C-repeat/DRE, a cis-acting DNA regulatory element that stimulates transcription in response to low temperature and water deficit. Proc. Natl. Acad. Sci. USA 94: 1035-1040.
Tahtiharju, S. and Palva, T. 2001. Antisense inhibition of protein phosphatase 2C accelerates cold acclimation in Arabidopsis thaliana. Plant J. 26: 461-470.
Thomashow, M.F. 1999. Plant cold acclimation, freezing tolerance genes and regulatory mechanisms. Annu. Rev. Plant Physiol. Plant Mol. Biol. 50: 571-599.
Walker, A.R., Davison, P.A., Bolognesi-Winfield, A.C., James, C.M., Srinivasan, N., Blundell, T.L., Esch, J.J., Marks, M.D., and Gray, J.C. 1999. The TRANSPARENT TESTA GLABRA1 locus, which regulates trichome differentiation and anthocyanin biosynthesis in Arabidopsis, encodes a WD40-repeat protein. Plant Cell 11: 1337-1349.
Xin, Z., and J. Browse 1998. eskimol mutants of Arabidopsis are constitutively freezing-tolerant. Proc. Natl. Acad. Sci. USA 95: 7799-7804.
Yamaguchi-Shinozaki, K., and Shinozaki, K. 1994. A novel cis-acting element in an Arabidopsis gene is involved in responsiveness to drought, low-temperature, or high-salt stress. Plant Cell 6: 251-264.

## Claims

1. A method of increasing cold acclimation of a plant in need thereof, comprising introducing into said plant a polynucleotide which encodes a protein comprising the amino acid sequence of SEQ ID NO:2.

2. A method of increasing cold acclimation of a plant in need thereof, comprising introducing the polynucleotide of SEQ ID NO:1 into said plant.

3. A method of increasing cold acclimation of a plant in need thereof, comprising enhancing the expression of the *ICE1* gene of SEQ ID NO:1 in said plant.

4. A method of increasing cold acclimation in a plant, comprising overexpressing an *ICE1* transcriptional activator in the plant, which *ICE1* transcriptional activator is chosen among:
an *ICE1* transcriptional activator having the amino acid sequence of SEQ ID NO:2 or an amino acid sequence which is at least 70% identical to SEQ ID NO:2 ;
an *ICE1* transcriptional activator encoded by a nucleic acid having the sequence of SEQ ID NO:1 or a sequence which is at least -70% identical to SEQ ID NO:1 ;
an *ICE1* transcriptional activator encoded by a nucleic acid which hybridizes under stringent conditions to the complement of SEO ID NO:1, wherein said stringent conditions comprise washing in 5X SSC at a temperature of from 50 to 68°C.

5. The method of Claim 4, wherein the *ICE1* transcriptional activator has the amino acid sequence of SED ID NO:2.

6. The method of Claim 5, wherein the *ICE1* transcriptional activator is encoded by a nucleic acid having the sequence of SEQ ID NO:1.

7. The method of Claim 4, wherein the *ICE1* transcriptional activator is encoded by a nucleic acid which has a sequence which is at least 70% identical to SEQ ID NO:1.

8. The method of Claim 4, wherein the *ICE1* transcriptional activator is encoded by a nucleic acid which has a sequence which is at least 90% identical to SEQ ID NO:1.

9. The method of Claim 4, wherein the ICE1 transcriptional activator is encoded by a nucleic acid which hybridizes under stringent conditions to the complement of SEQ ID NO:1, wherein said stringent conditions comprise washing in 5X SSC at a temperature of from 50 to 68°C.

10. The method of Claim 4, wherein the amino acid sequence of the *ICE1* transcriptional activator has an identity of at least 80% with SEQ ID NO:2.

11. The method of Claim 4, wherein the amino acid sequence of the *ICE1* transcriptional activator has an identity of at least 90% with SEQ ID NO:2.

12. The method of Claim 4, wherein the plant is *Arabidopsis thalania.*

13. The method of Claim 4, wherein the plant is selected from the group consisting of wheat, corn, peanut cotton, oat and soybean.

14. The method of Claim 4, wherein the plant has an increased expression of one or more additional transcription factors selected from the group consisting of a CBF transcription factor and a DREB 1 transcription factor.

15. The method of Claim 4, wherein the plant has an increased expression of one or more cold-responsive genes.

16. The method of Claim 15, wherein the cold responsive genes encode a protein selected from the group consisting of an enzyme involved in respiration of carbohydrates, an enzyme involved in metabolism of carbohydrates, an enzyme involved in respiration of lipids, an enzyme involved in metabolism of lipids, an enzyme involved in respiration of phenylpropanoids, an enzyme involved in metabolism of phenylpropanoids, an enzyme involved in respiration of antioxidants, an enzyme involved in metabolism of antioxidants, a molecular chaperone, an antifreeze protein, and a protein involved in tolerance to the dehydration caused by freezing.

17. The method of Claim 4, wherein the plant is transformed with a vector encoding the *ICE1* transcriptional activator, which *ICE1* transcriptional activator is chosen among:
an *ICE1* transcriptional activator having the amino acid sequence of SEQ ID NO:2 or an amino acid sequence which is at least 70% identical to SEQ ID NO:2 ;
an *ICE1* transcriptional activator encoded by a nucleic acid having the sequence of SEQ ID NO:1 or a sequence which is at least 70% identical to SEQ ID NO:1 ;
an *ICE1* transcriptional activator encoded by a nucleic acid which hybridizes under stringent conditions to the complement of SEQ ID NO:1, wherein said stringent conditions comprise washing in 5X SSC at a temperature of from 50 to 68°C.

18. The method of Claim 17, wherein the *ICE1* transcriptional activator has the amino acid sequence of SEQ ID NO:2.

19. The method of Claim 17, wherein *ICE1* transcriptional activator is encoded by a nucleic acid having the sequence of SEQ ID NO:1.

20. The method of Claim 17, wherein the *ICE1* transcriptional activator is encoded by a nucleic acid which has a sequence which is at least 70% identical to SEQ ID NO:1.

21. The method of Claim 17, wherein the *ICE1* transcriptional activator is encoded by a nucleic acid which has a sequence which is at least 90% identical to SEQ ID NO:1.

22. The method of Claim 17, wherein the *ICEI* transcriptional activator is encoded by a nucleic acid which hybridizes under stringent conditions to the complement of SEQ ID NO:1, wherein said stringent conditions comprise washing in 5X SSC at a temperature of from 50 to 68°C.

23. The method of Claim 17, wherein the amino acid sequence of *ICE1* transcriptional activator has an identity of at least 80% with SEQ ID NO:2.

24. The method of Claim 17, wherein the amino acid sequence of the *ICE1* transcriptional activator has an identity of at least 90% with SEQ ID NO:2.

25. The method of Claim 17, wherein the plant is *Arabidopsis thalania.*

26. The method of Claim 17, wherein the plant is selected from the group consisting of wheat, corn, peanut cotton, oat, and soybean.

27. The method of Claim 17, wherein the plant has an increased expression of one or more additional transcription factors selected from the group consisting of a CBF transcription factor and a DREB 1 transcription factor.

28. The method of Claim 17, wherein the plant has an increased expression of one or more cold-responsive genes.

29. The method of Claim 28, wherein the cold responsive genes encode a protein selected from the group consisting of an enzyme involved in respiration of carbohydrates, an enzyme involved in metabolism of carbohydrates, an enzyme involved in respiration of lipids, an enzyme involved in metabolism of lipids, an enzyme involved in respiration of phenylpropanoids, an enzyme involved in metabolism of phenylpropanoids, an enzyme involved in respiration of antioxidants, an enzyme involved in metabolism of antioxidants, a molecular chaperone, an antifreeze protein, and a protein involved in tolerance to the dehydration caused by freezing.

30. A method of enhancing expression of one or more cold-responsive genes in a plant cell, comprising transforming the plant with a vector which encodes an *ICE1* transcriptional activator, in the plant, which *ICE1* transcriptional activator is chosen among:
an *ICE1* transcriptional activator having the amino acid sequence of SEQ ID NO:2 or an amino acid sequence which is at least 70% identical to SEQ ID NO:2 ;
an *ICE1* transcriptional activator encoded by a nucleic acid having the sequence of SEQ ID NO:1 or a sequence which is at least 70% identical to SEQ ID NO:1 ;
an *ICE1* transcriptional activator encoded by a nucleic acid which hybridizes under stringent conditions to the complement of SEQ ID NO:1, wherein said stringent conditions comprise washing in 5X SSC at a temperature of from 50 to 68°C, and
wherein the plant has increased cold acclimation.

31. The method of Claim 30, wherein the *ICE1* transcriptional activator has the amino acid sequence of SEQ ID NO:2.

32. The method of Claim 30, wherein the *ICE1* transcriptional activator ts encoded by a nucleic acid having the sequence of SEQ ID NO: 1.

33. The method of Claim 30, wherein the *ICE1* transcriptional activator is encoded by a .nucleic acid which has a sequence which is at least 70% identical to SEQ ID NO:1.

34. The method of Claim 30, wherein the *ICE1* transcriptional activator is encoded by a nucleic acid which has a sequence which is at least 90% identical to SEQ ID NO:1.

35. The method of Claim 3.0, wherein the *ICE1* transcriptional activator is encoded by a nucleic acid which hybridizes under stringent conditions to the complement of SEQ ID NO:1, wherein said stringent conditions comprise washing in 5X SSC at a temperature of from 50 to 68°C.

36. The method of Claim 30, wherein the amino acid sequence of the *ICE1* transcriptional activator has an identify of at least 80% with SEQ ID NO:2.

37. The method of Claim 30, wherein the amino acid sequence of the *ICE1* transcriptional activator has an identify of at least 90% with SEQ ID NO:2.

38. The method of Claim 30, wherein the plant cell is *Arabidopsis thalania.*

39. The method of Claim 30, wherein the plant cell is selected from the group consisting of wheat, corn, peanut, cotton, oat, and soybeam.

## Patentansprüche

1. Verfahren zum Verbessern der Kälteakklimatisierung einer Pflanze, die dies benötigt, umfassend das Einschleusen eines Polynukleotids in diese Pflanze, das für ein Protein codiert, das die Aminosäuresequenz von SEQ ID NO: 2 umfasst.

2. Verfahren zum Verbessern der Kälteakklimatisierung einer Pflanze, die dies benötigt, umfassend das Einschleusen des Polynukleotids von SEQ ID NO: 1 in die Pflanze.

3. Verfahren zum Verbessern der Kälteakklimatisierung einer Pflanze, die dies benötigt, umfassend die Steigerung der Expression des *ICE1*-Gens von SEQ ID NO: 1 in der Pflanze.

4. Verfahren zum Verbessern der Kälteakklimatisierung in einer Pflanze, umfassend die Überexpression eines *ICE1*-Transkriptionsaktivators in der Pflanze, wobei der *ICE1*-Transkriptionsaktivator ausgewählt ist aus:
einem *ICE1*-Transkriptionsaktivator mit der Aminosäuresequenz von SEQ ID NO: 2 oder einer Aminosäuresequenz, die wenigstens 70 % mit SEQ ID NO: 2 identisch ist;
einem *ICE1*-Transkriptionsaktivator, der von einer Nukleinsäure mit der Sequenz von SEQ ID NO: 1 oder einer Sequenz, die wenigstens 70 % identisch mit SEQ ID NO: 1 ist, codiert wird;
einem *ICE1*-Transkriptionsaktivator, der von einer Nukleinsäure codiert wird, die unter stringenten Bedingungen mit dem Komplementärstrang von SEQ ID NO: 1 hybridisiert, wobei die stringenten Bedingungen Waschen in 5 x SSC bei einer Temperatur von 50 bis 68 °C umfassen.

5. Verfahren nach Anspruch 4, wobei der *ICE1*-Transkriptionsaktivator die Aminosäuresequenz von SEQ ID NO: 2 hat.

6. Verfahren nach Anspruch 5, wobei der *ICE1*-Transkriptionsaktivator von einer Nukleinsäure mit der Sequenz von SEQ ID NO: 1 codiert wird.

7. Verfahren nach Anspruch 4, wobei der *ICE1*-Transkriptionsaktivator von einer Nukleinsäure codiert wird, die eine Sequenz hat, die wenigstens 70 % identisch mit SEQ ID NO: 1 ist.

8. Verfahren nach Anspruch 4, wobei der *ICE1*-Transkriptionsaktivator von einer Nukleinsäure codiert wird, die eine Sequenz hat, die wenigstens 90 % identisch mit SEQ ID NO: 1 ist.

9. Verfahren nach Anspruch 4, wobei der *ICE1*-Transkriptionsaktivator von einer Nukleinsäure codiert wird, die unter stringenten Bedingungen mit dem Komplementärstrang von SEQ ID NO: 1 hybridisiert, wobei die stringenten Bedingungen Waschen in 5 x SSC bei einer Temperatur von 50 bis 68 °C umfassen.

10. Verfahren nach Anspruch 4, wobei die Aminosäuresequenz des *ICE1-*Transkriptionsaktivators eine Identität von wenigstens 80 % mit SEQ ID NO: 2 hat.

11. Verfahren nach Anspruch 4, wobei die Aminosäuresequenz des *ICE1-*Transkriptionsaktivators eine Identität von wenigstens 90 % mit SEQ ID NO: 2 hat.

12. Verfahren nach Anspruch 4, wobei die Pflanze *Arabidopsis thalania* ist.

13. Verfahren nach Anspruch 4, wobei die Pflanze ausgewählt ist aus der Gruppe bestehend aus Weizen, Mais, Erdnuss, Baumwolle, Hafer und Sojabohne.

14. Verfahren nach Anspruch 4, wobei die Pflanze eine erhöhte Expression ein oder mehrerer weiterer Transkriptionsfaktoren zeigt, ausgewählt aus der Gruppe bestehend aus einem CBF-Transkriptionsfaktor und einem DREB1-Transkriptionsfaktor.

15. Verfahren nach Anspruch 4, wobei die Pflanze eine erhöhte Expression ein oder mehrerer kälteinduzierbarer Gene zeigt.

16. Verfahren nach Anspruch 15, wobei die kälteinduzierbaren Gene für ein Protein codieren, das ausgewählt ist aus der Gruppe bestehend aus einem Enzym, das an Kohlenhydratrespiration beteiligt ist, einem Enzym, das am Kohlenhydratmetabolismus beteiligt ist, einem Enzym, das an Lipidrespiration beteiligt ist, einem Enzym, das am Lipidmetabolismus beteiligt ist, einem Enzym, das an Phenylpropanoidrespiration beteiligt ist, einem Enzym, das am Phenylpropanoidmetabolismus beteiligt ist, einem Enzym, das an Antioxidansrespiration beteiligt ist, einem Enzym, das am Metabolismus von Antioxidanzien beteiligt ist, einem molekularen Chaperon, einem Frostschutzprotein und einem Protein, das an Toleranz gegen Dehydrierung als Folge von Frost beteiligt ist.

17. Verfahren nach Anspruch 4, wobei die Pflanze mit einem Vektor transformiert wird, der für den *ICE1-*Transkriptionsaktivator codiert, wobei der *ICE1-*Transkriptionsaktivator ausgewählt ist aus:
einem *ICE1-*Transkriptionsaktivator mit der Aminosäuresequenz von SEQ ID NO: 2 oder einer Aminosäuresequenz, die wenigstens 70 % mit SEQ ID NO: 2 identisch ist;
einem *ICE1*-Transkriptionsaktivator, der von einer Nukleinsäure mit der Sequenz von SEQ ID NO: 1 oder einer Sequenz, die wenigstens 70 % identisch mit SEQ ID NO: 1 ist, codiert wird;
einem *ICE1*-Transkriptionsaktivator, der von einer Nukleinsäure codiert wird, die unter stringenten Bedingungen mit dem Komplementärstrang von SEQ ID NO: 1 hybridisiert, wobei die stringenten Bedingungen Waschen in 5 x SSC bei einer Temperatur von 50 bis 68 °C umfassen.

18. Verfahren nach Anspruch 17, wobei der *ICE1*-Transkriptionsaktivator die Aminosäuresequenz von SEQ ID NO: 2 hat.

19. Verfahren nach Anspruch 17, wobei der *ICE1*-Transkriptionsaktivator von einer Nukleinsäure mit der Sequenz von SEQ ID NO: 1 codiert wird.

20. Verfahren nach Anspruch 17, wobei der *ICE1*-Transkriptionsaktivator von einer Nukleinsäure codiert wird, die eine Sequenz hat, die wenigstens 70 % identisch mit SEQ ID NO: 1 ist.

21. Verfahren nach Anspruch 17, wobei der *ICE1*-Transkriptionsaktivator von einer Nukleinsäure codiert wird, die eine Sequenz hat, die wenigstens 90 % identisch mit SEQ ID NO: 1 ist.

22. Verfahren nach Anspruch 17, wobei der *ICE1*-Transkriptionsaktivator von einer Nukleinsäure codiert wird, die unter stringenten Bedingungen mit dem Komplementärstrang von SEQ ID NO: 1 hybridisiert, wobei die stringenten Bedingungen Waschen in 5 x SSC bei einer Temperatur von 50 bis 68 °C umfassen.

23. Verfahren nach Anspruch 17, wobei die Aminosäuresequenz des *ICE1-*Transkriptionsaktivators eine Identität von wenigstens 80 % mit SEQ ID NO: 2 hat.

24. Verfahren nach Anspruch 17, wobei die Aminosäuresequenz des *ICE1-*Transkriptionsaktivators eine Identität von wenigstens 90 % mit SEQ ID NO: 2 hat.

25. Verfahren nach Anspruch 17, wobei die Pflanze *Arabidopsis thalania* ist.

26. Verfahren nach Anspruch 17, wobei die Pflanze ausgewählt ist aus der Gruppe bestehend aus Weizen, Mais, Erdnuss, Baumwolle, Hafer und Sojabohne.

27. Verfahren nach Anspruch 17, wobei die Pflanze eine erhöhte Expression ein oder mehrerer weiterer Transkriptionsfaktoren zeigt, ausgewählt aus der Gruppe bestehend aus einem CBF-Transkriptionsfaktor und einem DREB1-Transkriptionsfaktor.

28. Verfahren nach Anspruch 17, wobei die Pflanze eine erhöhte Expression ein oder mehrerer kälteinduzierbarer Gene zeigt.

29. Verfahren nach Anspruch 28, wobei die kälteinduzierbaren Gene für ein Protein codieren, das ausgewählt ist aus der Gruppe bestehend aus einem Enzym, das an Kohlenhydratrespiration beteiligt ist, einem Enzym, das am Kohlenhydratmetabolismus beteiligt ist, einem Enzym, das an Lipidrespiration beteiligt ist, einem Enzym, das am Lipidmetabolismus beteiligt ist, einem Enzym, das an Phenylpropanoidrespiration beteiligt ist, einem Enzym, das am Phenylpropanoidmetabolismus beteiligt ist, einem Enzym, das an Antioxidansrespiration beteiligt ist, einem Enzym, das am Metabolismus von Antioxidanzien beteiligt ist, einem molekularen Chaperon, einem Frostschutzprotein und einem Protein, das an Toleranz gegen Dehydrierung als Folge von Frost beteiligt ist.

30. Verfahren zum Erhöhen der Expression ein oder mehrerer kälteinduzierbarer Gene in einer Pflanzenzelle, umfassend Transformieren der Pflanze mit einem Vektor, der für einen *ICE1*-Transkriptionsaktivator in der Pflanze codiert, wobei der *ICE1*-Transkriptionsaktivator ausgewählt ist aus:
einem *ICE1*-Transkriptionsaktivator mit der Aminosäuresequenz von SEQ ID NO: 2 oder einer Aminosäuresequenz, die wenigstens 70 % mit SEQ ID NO: 2 identisch ist;
einem *ICE1*-Transkriptionsaktivator, der von einer Nukleinsäure mit der Sequenz von SEQ ID NO: 1 oder einer Sequenz, die wenigstens 70 % identisch mit SEQ ID NO: 1 ist, codiert wird;
einem *ICE1*-Transkriptionsaktivator, der von einer Nukleinsäure codiert wird, die unter stringenten Bedingungen mit dem Komplementärstrang von SEQ ID NO: 1 hybridisiert, wobei die stringenten Bedingungen Waschen in 5 x SSC bei einer Temperatur von 50 bis 68 °C umfassen, und wobei die Pflanze verbesserte Kälteakklimatisierung zeigt.

31. Verfahren nach Anspruch 30, wobei der *ICE1*-Transkriptionsaktivator die Aminosäuresequenz von SEQ ID NO: 2 hat.

32. Verfahren nach Anspruch 30, wobei der *ICE1*-Transkriptionsaktivator von einer Nukleinsäure mit der Sequenz von SEQ ID NO: 1 codiert wird.

33. Verfahren nach Anspruch 30, wobei der *ICE1*-Transkriptionsaktivator von einer Nukleinsäure codiert wird, die eine Sequenz hat, die wenigstens 70 % identisch mit SEQ ID NO: 1 ist.

34. Verfahren nach Anspruch 30, wobei der *ICE1*-Transkriptionsaktivator von einer Nukleinsäure codiert wird, die eine Sequenz hat, die wenigstens 90 % identisch mit SEQ ID NO: 1 ist.

35. Verfahren nach Anspruch 30, wobei der *ICE1*-Transkriptionsaktivator von einer Nukleinsäure codiert wird, die unter stringenten Bedingungen mit dem Komplementärstrang von SEQ ID NO: 1 hybridisiert, wobei die stringenten Bedingungen Waschen in 5 x SSC bei einer Temperatur von 50 bis 68 °C umfassen.

36. Verfahren nach Anspruch 30, wobei die Aminosäuresequenz des *ICE1-*Transkriptionsaktivators eine Identität von wenigstens 80 % mit SEQ ID NO: 2 hat.

37. Verfahren nach Anspruch 30, wobei die Aminosäuresequenz des *ICE1-*Transkriptionsaktivators eine Identität von wenigstens 90 % mit SEQ ID NO: 2 hat.

38. Verfahren nach Anspruch 30, wobei die Pflanzenzelle *Arabidopsis thalania* ist.

39. Verfahren nach Anspruch 30, wobei die Pflanzenzelle ausgewählt ist aus der Gruppe bestehend aus Weizen, Mais, Erdnuss, Baumwolle, Hafer und Sojabohne.

## Revendications

1. Procédé d'augmentation de l'acclimatation au froid d'une plante en ayant besoin, comprenant l'introduction dans ladite plante d'un polynucléotide qui code une protéine comprenant la séquence d'acides aminés de SEQ ID NO:2.

2. Procédé d'augmentation de l'acclimatation au froid d'une plante en ayant besoin, comprenant l'introduction du polynucléotide de SEQ ID NO:1 dans ladite plante.

3. Procédé d'augmentation de l'acclimatation au froid d'une plante en ayant besoin, comprenant l'accroissement de l'expression du gène *ICE1* de SEQ ID NO:1 dans ladite plante.

4. Procédé d'augmentation de l'acclimatation au froid chez une plante, comprenant la sur-expression d'un activateur transcriptionnel *ICE1* dans la plante, cet activateur transcriptionnel *ICE1* étant choisi parmi :
un activateur transcriptionnel *ICE1* ayant la séquence d'acides aminés de SEQ ID NO:2 ou une séquence d'acides aminés qui est identique à au moins 70 % à SEQ ID NO:2 ;
un activateur transcriptionnel *ICE1* codé par un acide nucléique ayant la séquence de SEQ ID NO:1 ou une séquence qui est identique à au moins 70 % à SEQ ID NO:1 ;
un activateur transcriptionnel *ICE1* codé par un acide nucléique qui s'hybride dans des conditions stringentes au complément de SEQ ID NO:1, dans lequel lesdites conditions stringentes comprennent le lavage dans SSC 5X à une température de 50 à 68 °C.

5. Procédé selon la revendication 4, dans lequel l'activateur transcriptionnel *ICE1* a la séquence d'acides aminés de SEQ ID NO:2.

6. Procédé selon la revendication 5, dans lequel l'activateur transcriptionnel *ICE1* est codé par un acide nucléique ayant la séquence de SEQ ID NO:1.

7. Procédé selon la revendication 4, dans lequel l'activateur transcriptionnel *ICE1* est codé par un acide nucléique qui a une séquence qui est identique à au moins 70 % à SEQ ID NO:1.

8. Procédé selon la revendication 4, dans lequel le vecteur transcriptionnel *ICE1* est codé par un acide nucléique qui a une séquence qui est identique à au moins 90 % à SEQ ID NO:1.

9. Procédé selon la revendication 4, dans lequel l'activateur transcriptionnel *ICE1* est codé par un acide nucléique qui s'hybride dans des conditions stringentes au complément de SEQ ID NO:1, dans lequel lesdites conditions stringentes comprennent le lavage dans SSC 5X à une température de 50 à 68 °C.

10. Procédé selon la revendication 4, dans lequel la séquence d'acides aminés de l'activateur transcriptionnel *ICE1* a une identité d'au moins 80 % avec SEQ ID NO:2.

11. Procédé selon la revendication 4, dans lequel la séquence d'acides aminés de l'activateur transcriptionnel *ICE1* a une identité d'au moins 90 % avec SEQ ID NO:2.

12. Procédé selon la revendication 4, dans lequel la plante est *Arabidopsis thalania.*

13. Procédé selon la revendication 4, dans lequel la plante est choisie dans le groupe constitué par le blé, le maïs, l'arachide, le coton, l'avoine et le soja.

14. Procédé selon la revendication 4, dans lequel la plante a une expression accrue d'un ou plusieurs facteurs de transcription supplémentaires choisis dans le groupe constitué par un facteur de transcription CBF et un facteur de transcription DREB1.

15. Procédé selon la revendication 4, dans lequel la plante a une expression accrue d'un ou plusieurs gènes réagissant au froid.

16. Procédé selon la revendication 15, dans lequel les gènes réagissant au froid codent une protéine choisie dans le groupe constitué par une enzyme intervenant dans la respiration des hydrates de carbone, une enzyme intervenant dans la métabolisme des hydrates de carbone, une enzyme intervenant dans la respiration des lipides, une enzyme intervenant dans le métabolisme des lipides, une enzyme intervenant dans la respiration des phénylpropanoïdes, une enzyme intervenant dans le métabolisme des phénylpropanoïdes, une enzyme intervenant dans la respiration des antioxydants, une enzyme intervenant dans le métabolisme des antioxydants, une molécule chaperonne, une protéine antigel et une protéine intervenant dans la tolérance à la déshydratation provoquée par une congélation.

17. Procédé selon la revendication 4, dans lequel la plante est transformée avec un vecteur codant l'activateur transcriptionnel *ICE1,* cet activateur transcriptionnel *ICE1* étant choisi parmi :
un activateur transcriptionnel *ICE1* ayant la séquence d'acides aminés de SEQ ID NO:2 ou une séquence d'acides aminés qui est identique à au moins 70 % à SEQ ID NO:2 ;
un activateur transcriptionnel *ICE1* codé par un acide nucléique ayant la séquence de SEQ ID NO:1 ou une séquence qui est identique à au moins 70 % à SEQ ID NO:1 ;
un activateur transcriptionnel *ICE1* codé par un acide nucléique qui s'hybride dans des conditions stringentes au complément de SEQ ID NO:1, dans lequel lesdites conditions stringentes comprennent le lavage dans SSC 5X à une température de 50 à 68 °C.

18. Procédé selon la revendication 17, dans lequel l'activateur transcriptionnel *ICE1* a la séquence d'acides aminés de SEQ ID NO:2.

19. Procédé selon la revendication 17, dans lequel l'activateur transcriptionnel *ICE1* est codé par un acide nucléique ayant la séquence de SEQ ID NO:1.

20. Procédé selon la revendication 17, dans lequel l'activateur transcriptionnel *ICE1* est codé par un acide nucléique qui a une séquence qui est identique à au moins 70 % à SEQ ID NO:1.

21. Procédé selon la revendication 17, dans lequel l'activateur transcriptionnel *ICE1* est codé par un acide nucléique qui a une séquence qui est identique à au moins 90 % à SEQ ID NO:1.

22. Procédé selon la revendication 17, dans lequel l'activateur transcriptionnel *ICE1* est codé par un acide nucléique qui s'hybride dans des conditions stringentes au complément de SEQ ID NO:1, dans lequel lesdites conditions stringentes comprennent le lavage dans SSC 5X à une température de 50 à 68 °C.

23. Procédé selon la revendication 17, dans lequel la séquence d'acides aminés de l'activateur transcriptionnel *ICE1* a une identité d'au moins 80 % avec SEQ ID NO:2.

24. Procédé selon la revendication 17, dans lequel la séquence d'acides aminés de l'activateur transcriptionnel *ICE1* a une identité d'au moins 90 % avec SEQ ID NO:2.

25. Procédé selon la revendication 17, dans lequel la plante est *Arabidopsis thalania.*

26. Procédé selon la revendication 17, dans lequel la plante est choisie dans le groupe constitué par le blé, le maïs, l'arachide, le coton, l'avoine et le soja.

27. Procédé selon la revendication 17, dans lequel la plante a une expression accrue d'un ou plusieurs facteurs de transcription supplémentaires choisis dans le groupe constitué par un facteur de transcription CBF et un facteur de transcription DREB1.

28. Procédé selon la revendication 17, dans lequel la plante a une expression accrue d'un ou plusieurs gènes réagissant au froid.

29. Procédé selon la revendication 28, dans lequel les gènes réagissant au froid codent une protéine choisie dans le groupe constitué par une enzyme intervenant dans la respiration des hydrates de carbone, une enzyme intervenant dans la métabolisme des hydrates de carbone, une enzyme intervenant dans la respiration des lipides, une enzyme intervenant dans le métabolisme des lipides, une enzyme intervenant dans la respiration des phénylpropanoïdes, une enzyme intervenant dans le métabolisme des phénylpropanoïdes, une enzyme intervenant dans la respiration des antioxydants, une enzyme intervenant dans le métabolisme des antioxydants, une molécule chaperonne, une protéine antigel et une protéine intervenant dans la tolérance à la déshydratation provoquée par une congélation.

30. Procédé d'activation de l'expression d'un ou plusieurs gènes réagissant au froid dans une cellule de plante, comprenant la transformation de la plante avec un vecteur qui code un activateur transcriptionnel *ICE1,* dans la plante, cet activateur transcriptionnel *ICE1* étant choisi parmi :
un activateur transcriptionnel *ICE1* ayant la séquence d'acides aminés de SEQ ID NO:2 ou une séquence d'acides aminés qui est identique à au moins 70 % à SEQ ID NO:2 ;
un activateur transcriptionnel *ICE1* codé par un acide nucléique ayant la séquence de SEQ ID NO:1 ou une séquence qui est identique à au moins 70 % à SEQ ID NO:1 ;
un activateur transcriptionnel *ICE1* codé par un acide nucléique qui s'hybride dans des conditions stringentes au complément de SEQ ID NO:1, dans lequel lesdites conditions stringentes comprennent le lavage dans SSC 5X à une température de 50 à 68 °C, et
dans lequel la plante a une acclimatation au froid accrue.

31. Procédé selon la revendication 30, dans lequel l'activateur transcriptionnel *ICE1* a la séquence d'acides aminés de SEQ ID NO:2.

32. Procédé selon la revendication 30, dans lequel l'activateur transcriptionnel *ICE1* est codé par un acide nucléique ayant la séquence de SEQ ID NO:1.

33. Procédé selon la revendication 30, dans lequel l'activateur transcriptionnel *ICE1* est codé par un acide nucléique qui a une séquence qui est identique à au moins 70 % à SEQ ID NO:1.

34. Procédé selon la revendication 30, dans lequel l'activateur transcriptionnel *ICE1* est codé par un acide nucléique qui a une séquence qui est identique à au moins 90 % à SEQ ID NO:1.

35. Procédé selon la revendication 30, dans lequel l'activateur transcriptionnel *ICE1* est codé par un acide nucléique qui s'hybride dans des conditions stringentes au complément de SEQ ID NO:1, dans lequel lesdites conditions stringentes comprennent le lavage dans SSC 5X à une température de 50 à 68 °C.

36. Procédé selon la revendication 30, dans lequel la séquence d'acides aminés de l'activateur transcriptionnel *ICE1* a une identité d'au moins 80 % avec SEQ ID NO:2.

37. Procédé selon la revendication 30, dans lequel la séquence d'acides aminés de l'activateur transcriptionnel. *ICE1* a une identité d'au moins 90 % avec SEQ ID NO:2.

38. Procédé selon la revendication 30, dans lequel la cellule de plante est *Arabidopsis thalania.*

39. Procédé selon la revendication 30, dans lequel la cellule du plante est choisie dans le groupe constitué par le blé, le maïs, l'arachide, le coton, l'avoine et le soja.
